(19) Europäisches Patentamt / European Patent Office / Office européen des brevets

(11) **EP 3 064 232 B1**

(12) **EUROPEAN PATENT SPECIFICATION**

(45) Date of publication and mention
of the grant of the patent:
**09.12.2020   Bulletin 2020/50**

(51) Int Cl.:
*A61L 31/10* *(2006.01)*          *A61L 31/02* *(2006.01)*
*A61F 2/82* *(2013.01)*          *A61F 2/915* *(2013.01)*

(21) Application number: **14857514.5**

(22) Date of filing: **31.10.2014**

(86) International application number:
**PCT/CN2014/090107**

(87) International publication number:
**WO 2015/062546 (07.05.2015 Gazette 2015/18)**

(54) **ABSORBABLE IRON ALLOY STENT**

ABSORBIERBARER EISENLEGIERUNGSSTENT

ENDOPROTHÈSE D'ALLIAGE DE FER ABSORBABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.10.2013   CN 201310533326**

(43) Date of publication of application:
**07.09.2016   Bulletin 2016/36**

(73) Proprietor: **Biotyx Medical (Shenzhen) Co., Ltd.
Shenzhen, Guangdong 518000 (CN)**

(72) Inventors:
• **ZHANG, Deyuan
Shenzhen
Guangdong 518000 (CN)**
• **SUN, Hongtao
Shenzhen
Guangdong 518000 (CN)**
• **CHEN, Liping
Shenzhen
Guangdong 518000 (CN)**

(74) Representative: **Prinz & Partner mbB
Patent- und Rechtsanwälte
Rundfunkplatz 2
80335 München (DE)**

(56) References cited:
WO-A1-2013/025535          CN-A- 101 337 090
CN-A- 102 228 721          CN-A- 102 228 721
US-A1- 2005 209 680          US-A1- 2008 033 539
US-A1- 2009 192 594

• RYAN D. ALEXY ET AL: "Materials and
Manufacturing Technologies Available for
Production of a Pediatric Bioabsorbable Stent",
BIOMED RESEARCH INTERNATIONAL, vol. 2013,
6 August 2013 (2013-08-06), pages 1-11,
XP055378811, ISSN: 2314-6133, DOI:
10.1155/2013/137985
• PEUSTER M ET AL: "A NOVEL APPROACH TO
TEMPORARY STENTING: DEGRADABLE
CARDIOVASCULAR STENTS PRODUCED FROM
CORRODIBLE METAL-RESULTS 6-18 MONTHS
AFTER IMPLANTATION INTO NEW ZEALAND
WHITE RABBITS", H, BMJ, LONDON, GB, vol. 86,
no. 5, 1 November 2001 (2001-11-01), pages
563-569, XP009009646, ISSN: 1355-6037, DOI:
10.1136/HEART.86.5.563
• GARG S ET AL: "Biodegradable and
non-biodegradable stents", MINERVA
CARDIOANGIOLOGICA, EDIZIONI MINERVA
MEDICA, TORINO, IT, vol. 57, no. 5, 1 October
2009 (2009-10-01), pages 537-565, XP008184802,
ISSN: 0026-4725

## Description

Technical field

The present invention relates to a degradable implantable medical device, and particularly relates to an absorbable iron-based alloy stent capable of degrading rapidly and controllably within a predetermined period.

Background art

At present, the implantable medical devices are usually made from metals and their alloys, ceramics, polymers and the related composite materials, wherein the metal-based implantable medical devices are particularly popular because of their superior mechanical properties, such as high strength, and high toughness.

Iron as an important element in the human body, is involved in many biochemical processes, such as in the delivery of oxygen. Easily corrosive pure iron stents each of the shape similar to that of a clinically used metal stent, made by Peuster, M. et al. through a laser engraving method, were respectively implanted to the descending aortas of 16 New Zealand rabbits. The animal experimental results showed that there was no thrombosis complication within 6 to 18 months, and also no adverse events occurred. The pathological examination confirmed that there were no inflammation in local blood vessel walls and no obvious proliferation on smooth muscle cells, preliminarily indicating that the degradable iron stent is safe and reliable and has good application prospects. But the study also found that the corrosion rate of pure iron was relatively slow in vivo, which cannot meet the clinical degradation time requirement for the degradable stent, thus the corrosion rate of iron needed to be accelerated.

Various techniques for improving the corrosion rate of iron have been continuously developed, including alloying, changing the iron metallurgical structure, or coating of a degradable polyester coating layer on the surface of the iron-based alloy stent. For the method for increasing the corrosion rate of the iron-based material by the polyester, the literature disclosed that the degradable polyester coating would produce a product with a carboxyl group in the degradation process in the human body, so that the pH value of the local microenvironment near the stent implantation position dropped to form a local sub-acid environment, thereby the overpotential of hydrogen evolution reaction on the surface of the iron-based alloy substrate was reduced, and the hydrogen evolution corrosion was produced in the iron-based alloy substrate, thus producing an iron salt as a degradation product. The literature also indicated that the degradation process of iron-based alloy was accompanied by the oxygen-consuming corrosion process and the hydrogen evolution corrosion process, and because the highest oxygen-consuming corrosion rate of a solution in the local sub-acid environment is a constant value, it is difficult to improve the corrosion rate of the iron-based alloy by speeding up the oxygen-consuming corrosion rate. Namely, the literature believed that the corrosion rate of the iron-based alloy was improved only by the hydrogen evolution corrosion in the degradation of iron-based alloy. In addition, the literature only provided experimental data to indicate that the corrosion rate of iron-based alloy was increased under the action of polyester, and did not disclose the molecular weight and molecular weight distribution of the polymer, namely, did not disclose the match between the degradable polymer degradation and the iron-based alloy substrate corrosion. The literature also did not provide any experimental data to prove that the iron-based alloy stent can meet the clinical early mechanical property requirement after being implanted into the human body, and also did not disclose the corrosion period of the stent, so that whether the stent meets the clinical property requirement for the stent cannot be known by those skilled in the art.

In fact, a large amount of hydrogen produced by hydrogen evolution corrosion will cause the tissue tolerance risk such as formation of air embolism, so that the stent cannot be used clinically. Our early experiments showed that after nitrogen was introduced and oxygen was removed in the corrosion environment, the corrosion rate of iron-based alloy was greatly reduced, i.e., the hydrogen evolution corrosion indicated by the above-mentioned literature was not produced in the iron-based alloy in the body. If the corrosion rate of iron is too rapid, it is possible that the iron-based alloy stent at early stage (such as 3 months) after the implanting would not have sufficient structural integrity, and it is difficult to reach the radial support force required by the blood vessel clinically, so that the stent loses its clinical application value. Otherwise, if an increase in the corrosion rate of iron caused by the polymer is limited, the corrosion period of the iron-based alloy is longer, and it is difficult to meet the clinical degradation time requirement for the degradable stent. Therefore, it is necessary to adopt a specific degradable polymer that is matched with the iron-based alloy substrate, realizing the rapid and controllable corrosion of the iron-based alloy, thus obtaining an absorbable iron-based alloy stent capable of meeting the clinical requirements.

CN 102 228 721 discloses a degradable coronary stent by which an iron-based material is provided as a matrix and a surface of the matrix is covered with a macromolecular coating containing a degradable polyester.

Summary of the invention

The object to be solved by the present invention is to provide an absorbable iron-based alloy stent capable of degrading rapidly and controllably within a predetermined period after being implanted into the body in order to overcome the shortcomings of the prior art.

According to one solution of the present invention, an absorbable iron-based alloy stent is provided as defined in claim 1. The absorbable iron-based alloy stent, comprises an iron-based alloy substrate and a degradable polyester in contact with the surface of the substrate, wherein the degradable polyester has a weight average molecular weight in the range of 20,000 to 1,000,000 and a polydispersity index more than or equal to 1.2 and lessthan or equal to 30, or more than 1.0 and less than 1.2, or more than 30 and less than or equal to 50,and wherein the surface of the iron-based alloy substrate is coated with degradable polyester in the form of a coating layer (3),

wherein when a wall thickness of the iron-based alloy substrate is more than or equal to 30 μm and less than 50 μm, a thickness of the degradable polyester coating layer (3) is more than or equal to 3 μm and less than 5 μm, or more than or equal to 5 μm and less than 10 μm, or more than or equal to 10 μm and less than 15 μm, or more than or equal to 15 μm and less than or equal to 20 μm;

or when the wall thickness of the iron-based alloy substrate is more than or equal to 50 μm and less than 100 μm, the thickness of the degradable polyester coating layer (3) is more than or equal to 5 μm and less than 10 μm, or more than or equal to 10 μm and less than 15 μm, or more than or equal to15 μm and less than 20 μm, or more than or equal to 20 μm and less than or equal to 25 μm;

or when the wall thickness of the iron-based alloy substrate is more than or equal to 100 μm and less than 200 μm, the thickness of the degradable polyester coating layer (3) is more than or equal to 10 μm and less than 15 μm, or more than or equal to 15 μm and less than 20 μm, or more than or equal to 20 μm and less than 25 μm, or more than or equal to 25 μm and less than or equal to 35 μm;

or when the wall thickness of the iron-based alloy substrate is more than or equal to 200 μm and less than or equal to 300 μm, and the thickness of the degradable polyester coating layer (3) is more than or equal to 10 μm and less than 15 μm, or more than or equal to 15 μm and less than 20 μm, or more than or equal to 20 μm and less than 25 μm, or more than or equal to 25 μm or less than 35 μm, or more than or equal to 35 μm and less than or equal to 45 μm.

In another solution according to the present invention, an absorbable iron-based alloy stent in provided as defined in claim 11. The stent comprises an iron-based alloy substrate and a degradable polymer in contact with the surface of the substrate, wherein the degradable polymer has a weight average molecular weight more than or equal to 20,000 and less than or equal to 1,000,000 and a polydispersity index of more than 1.0 and less than or equal to 50, and the degradable polymer is capable of producing a carboxyl group after the iron-based alloy stent is implanted into the body, and wherein the surface of the iron-based alloy substrate is coated with the degradable polymer in the form of a coating layer (3),

wherein when a wall thickness of the iron-based alloy substrate is more than or equal to 30 μm and less than 50 μm, a thickness of the degradable polymer coating layer (3) is more than or equal to 3 μm and less than 5 μm, or more than or equal to 5 μm and less than 10 μm, or more than or equal to 10 μm and less than 15 μm, or more than or equal to 15 μm and less than or equal to 20 μm;

or when the wall thickness of the iron-based alloy substrate is more than or equal to 50 μm and less than 100 μm, the thickness of the degradable polymer coating layer (3) is more than or equal to 5 μm and less than 10 μm, or more than or equal to 10 μm and less than 15 μm, or more than or equal to15 μm and less than 20 μm, or more than or equal to 20 μm and less than or equal to 25 μm;

or when the wall thickness of the iron-based alloy substrate is more than or equal to 100 μm and less than 200 μm, the thickness of the degradable polymer coating layer (3) is more than or equal to 10 μm and less than 15 μm, or more than or equal to 15 μm and less than 20 μm, or more than or equal to 20 μm and less than 25 μm, or more than or equal to 25 μm and less than or equal to 35 μm;

or when the wall thickness of the iron-based alloy substrate is more than or equal to 200 μm and less than or equal to 300 μm, the thickness of the degradable polymer coating layer (3) is more than or equal to 10 μm and less than 15 μm, or more than or equal to 15 μm and less than 20 μm, or more than or equal to 20 μm and less than 25 μm, or more than or equal to 25 μm and less than 35 μm, or more than or equal to 35 μm and less than or equal to 45μm.

In an embodiment of this solution, the degradable polymer may be a degradable polyester, a blend of the degradable polyester and a degradable polyanhydride, or a copolymer formed by copolymerizing monomers forming the degradable polyester and the degradable polyanhydride.

The iron-based alloy substrate may refer to a bare iron-based alloy stent, while the iron-based alloy substrate may be selected from pure iron or a medical iron-based alloy. At least one of nutrient elements and harmless elements in the human body, or less toxic elements, such as C, N, 0, S, P, Mn, Pd, Si, W, Ti, Co, Cr, Cu, and Re may be doped into the pure iron to form the medical iron-based alloy.

The numerical interval is in accordance with the mathematical knowledge, namely, [a, b] means more than or equal to a and less than or equal to b; (a, b] means more than a and less than or equal to b; [a, b) means more than or equal to a and less than b. The same symbols shall apply hereinafter without need of repetition.

The term "rapid" means that the degradable polyester can accelerate the corrosion of the iron-based alloy substrate, so that the iron-based alloy substrate can completely corrode within 5 years after being implanted into the body.

The term "controllable" means that the corrosion of the iron-based alloy substrate caused by the degradable polyester ensures that the iron-based alloy stent has good mechanical properties at early stage after being implanted into the human body, and also enables the stent to produce a small amount of hydrogen or no hydrogen. A small amount of hydrogen refers to an amount that is not sufficient to form a risk of air embolism clinically.

The term "complete corrosion" means that the mass loss rate W of the iron-based alloy stent is more than or equal to 90 percent.

The complete corrosion is characterized by a mass loss test of an animal experiment. The mass loss test is carried out by implanting an iron-based alloy stent with an iron-based alloy substrate (i.e., a bare stent excluding a degradable polymer) of which the mass is Mo into the abdominal aorta of a rabbit, cutting out the iron-based alloy stent implanted into an animal body and the tissue in which the iron-based alloy stent is placed at a predetermined observation point in time, soaking the tissue together with the stent in a solution of certain concentration (such as Imol/L of a sodium hydroxide solution) so that the tissue is digested, and then taking a stent strut out of the solution, putting the stent strut into a solution of a certain concentration (such as 3% of a tartaric acid solution, and/ or an organic solution) to be ultrasonically cleaned so that corrosion products on the surface of the stent are all stripped off or dissolved in the solution, taking the residual stent strut out of the solution, drying and weighing the stent strut to obtain the mass Mt. The mass loss rate W is expressed by a percentage of the weight loss difference value of the stent strut after corrosion and cleaning in weight of the iron-based alloy substrate, as shown in Formula 1-1:

$$W = \frac{(M_0 - M_t)}{M_0} \times 100\%$$

W - Mass loss rate

Mt Mass of the residual stent after corrosion

Mo - Mass of the iron-based alloy substrate

When the mass loss rate W of the stent is more than or equal to 90 percent, the iron-based alloy stent is deemed to be completely corroded.

The good mechanical properties obtained at early stage of implantation into the body are determined by specific clinical requirements. Generally, "early stage" refers to within 1 month, or 3 months, or 6 months after implantation into the body. The mechanical properties can be tested and verified by an animal experiment, and expressed by early OCT follow-up or a radial support force test. When the OCT follow-up is carried out, there is no obvious difference between the surrounding area of the stent and the surrounding area of the stent at the beginning of implanting, or when the radial support force test is carried out, the radial support force is more than 23.3 kPa (175 mm mercury column), indicating that the stent has good mechanical properties at early stage of implantation into the body.

Compared with the prior art, the iron-based alloy stent provided by the present invention does not produce hydrogen at all or only produces a small amount of hydrogen in the whole corrosion process, which is specifically tested and verified by means of an animal experiment. For example, the iron-based alloy stent is implanted into the abdominal aorta of a rabbit, and the periphery of the support strut is observed by a microscope at the same amplification factor at the predetermined observation point in time, such as 1 month, 3 months, 6 months, 2 years, 3 years or 5 years after the stent is implanted into the body; if few stent strut coating slightly bulges, the stent is deemed to produce a small amount of hydrogen during corrosion; if the stent strut uniformly corrodes, and there are no bubbles around the stent strut, the stent is deemed to not produce hydrogen at all in the corrosion process.

In the above-mentioned solutions, the weight average molecular weight of the degradable polyester is preferably more than or equal to 20,000 and less than 100,000, or more than or equal to 100,000 and less than 250,000, or more than or equal to 250,000 and less than 400,000, or more than or equal to 400,000 and less than 600,000, or more than or equal to 600,000 and less than or equal to 1,000,000.

In an embodiment of the solutions, the degradable polymer a degradable polyester, the degradable polyester is any one of the following: polylactic acid (PLA), polyglycolic acid (PGA), poly (butylene succinate)(PBS), poly (beta-hydroxybutyrate) (PHB), polycaprolactone (PCL), poly (ethyleneglycol adipate) (PEA), poly (lactic- co - glycolic acid) (PLGA), and poly (3-hydroxybutyrate-co-3-hydroxyvalerate) (PHBV).

In a further embodiment, the degradable polyester may comprise at least two kinds of the same type of degradable polyester polymers. The "same type" refers to a general term of polymers with the same structural unit (i.e., the monomers are the same) and different weight average molecular weights. The first kind of degradable polyester polymer has a weight average molecular weight of more than or equal to 20,000 and less than 100,000 and the second kind of degradable polyester polymer has a weight average molecular weight that is greater than or equal to 100,000 and less than or equal to 1,000,000. The mass ratio of the first kind of degradable polyester polymer to the second kind of degradable polyester polymer is in the range of 1:9 to 9:1. The same type of degradable polyester polymer is selected from the group consisting of polylactic acid (PLA), polyglycolic acid (PGA), poly(butylene succinate) (PBS), poly (beta-hydroxybutyrate) (PHB), polycaprolactone (PCL), poly(ethyleneglycol adipate) (PEA), poly(lactic-co-glycolic acid) (PLGA), or poly(3-hydroxybutyrate-co-3-hydroxyvalerate) (PHBV). The surface of the stent can be respectively coated with at least two kinds of the same type of degradable polyester polymers with different weight average molecular weights respectively, and can also be coated with the uniformly mixed degradable polyester polymers with different weight average molecular weights.

Furthermore, when the degradable polymer is a degradable polyester, the mass ratio of the first kind of degradable polyester polymer to the second kind of degradable polyester polymer may be in the range of 1:5 to 5:1.

In another embodiment, the degradable polyester comprises at least two kinds of degradable polyester polymers with high molecular weights, wherein the weight average molecular weights of at least two kinds of degradable polyester polymers with high molecular weights are more than or equal to 100,000 and less than 200,000, or more than or equal to 200,000 and less than 400,000, or more than or equal to 400,000 and less than 600,000, or more than or equal to 600,000 and less than or equal to 1,000,000.

In another embodiment, when the degradable polymer is a degradable polyester, the degradable polyester is a mixture of at least two of polylactic acid (PLA), polyglycolic acid (PGA), poly (butylene succinate) (PBS), poly (beta-hydroxybutyrate) (PHB), polycaprolactone (PCL), poly(ethyleneglycol adipate) (PEA), poly(lactic-co-glycolic acid) (PLGA), and poly(3-hydroxybutyrate-co-3-hydroxyvalerate) (PHBV), or a copolymer formed by copolymerizing at least two of monomers forming polylactic acid (PLA), polyglycolic acid (PGA), poly (butylene succinate)(PBS), poly (beta-hydroxybutyrate) (PHB), polycaprolactone (PCL), poly(ethyleneglycol adipate) (PEA), poly(lactic -co-glycolic acid) (PLGA), and poly(3-hydroxybutyrate-co-3-hydroxyvalerate) (PHBV). As an example, the degradable polyester may comprise polylactic acid (PLA) and poly (lactic-co-glycolic acid) (PLGA), wherein the weight average molecular weight of PLGA is more than or equal to 20,000 and less than 300,000, the weight average molecular weight of PLA is more than or equal to 20,000 and less than or equal to 1,000,000, and the content ratio of the two is in the range of 1:9 to 9:1.

In another embodiment, when the degradable polymer is a degradable polyester, the degradable polyester is a mixture of at least two kinds of degradable polyester polymers with different crystallinity, wherein the content of degradable polyester polymer with crystallinity in the range of 5% to 50% is in the range of 10% to 90% in percentage by mass. The degradable polyester polymer is selected from the group consisting of polylactic acid (PLA), polyglycolic acid (PGA), poly (butylene succinate)(PBS), poly (beta-hydroxybutyrate) (PHB), polycaprolactone (PCL), poly (ethyleneglycol adipate) (PEA), poly (lactic- co -glycolic acid) (PLGA), and poly (3-hydroxybutyrate-co-3-hydroxyvalerate) (PHBV).

At least two kinds of degradable polyester polymers with different crystallinity include the following: the degradable polyester may be a mixture of crystalline and non-crystalline degradable polyester polymers, or a blend of degradable polyester polymers with low crystallinity and high crystallinity. For example, the degradable polyester comprises polylactic acid (PLA) with crystallinity in the range of 5% to 50%, and the content of the polylactic acid (PLA) is in the range of 10% to 90% in percentage by mass. Preferably, the polylactic acid (PLA)may be poly (DL-lactic acid) or poly (L-lactic acid).

In the solution according to claim 11, the degradable polymer in one embodiment may be a mixture of a degradable polyester and a degradable polyanhydride, or a degradable copolymer formed by copolymerizing monomers forming the degradable polyester and the degradable polyanhydride, the degradable polyester and the degradable polyanhydride have weight average molecular weights more than or equal to 20,000 and less than or equal to 1,000,000, and polydispersity indexes of more than 1.0 and less than or equal to 50, the polyanhydride is selected from the group consisting of poly (1,3- bis(p-carboxyphenoxy) propane-sebacic acid) and poly (erucic acid dimer- sebacic acid) or poly (fumaric-sebacic acid), and the degradable polyester is any one of the following: polylactic acid (PLA), polyglycolic acid (PGA), poly(butylene succinate) (PBS), poly(beta-hydroxybutyrate) (PHB), polycaprolactone (PCL), poly(ethyleneglycol adipate) (PEA), poly(lactic-co-glycolic acid) (PLGA), and poly(3-hydroxybutyrate-co-3-hydroxyvalerate) (PHBV). The mass ratio of the degradable polyester to the polyanhydride is in the range of 1:9 to 9:1.

In another embodiment, in the mixture of the degradable polyester and the degradable polyanhydride, the content of degradable polyester or degradable polyanhydride with crystallinity in the range of 5% to 50% is in the range of 10% to 90% in percentage by mass, the degradable polyester polymer is selected from the group consisting of polylactic acid (PLA), polyglycolic acid (PGA), poly (butylene succinate)(PBS) and poly (beta-hydroxybutyrate) (PHB), polycaprolactone (PCL), poly (ethyleneglycol adipate) (PEA), poly (lactic- co -glycolic acid) (PLGA), and poly (3-hydroxybutyrate-co-3-hydroxyvalerate) (PHBV), and the polyanhydride is selected from the group consisting of poly (1, 3-bis(p-carboxyphenoxy) propane-sebacic acid) and poly(erucic acid dimer-sebacic acid) or poly(fumaric - sebacic acid).

In another embodiment, the degradable polyester or degradable polymer may also be in contact with the surface of the iron-based alloy substrate in the form of a non-coating layer, for example, the iron-based alloy substrate is provided with gaps or grooves, and the degradable polyester or the degradable polymer is arranged in the gaps or grooves; or the iron-based alloy substrate is provided with an inner cavity, and the degradable polyester or the degradable polymer is filled in the inner cavity. At least one of the non-coating contact form and the coating form in the above-mentioned example is selected. Namely, in the above-mentioned first to third technical solutions, the "surface" of "in contact with the surface of the substrate" not only refers to the outer surface, but also refers to all circumstances in which the degradable polyester or the degradable polymer has a contact point or contact surface with the iron-based alloy substrate.

In a further embodiment, the degradable polyester or the degradable polymer may also be mixed with an active drug, and the mass ratio of the degradable polyester or the degradable polymer to the drug is in the range of 0.1 to 20. The active drug may be a vascular proliferation inhibiting drug such as paclitaxel, rapamycin and their derivatives, or an antiplatelet drug selected from cilostazol, or an antithrombotic drug such as heparin, or an anti-inflammatory drug such as dexamethasone, or a mixture of the above-mentioned drugs. Furthermore, the mass ratio of the degradable polyester or the degradable polymer to the drug is in the range of 0.5 to 10.

Compared with the prior art, the specific degradable polymer used by the absorbable iron-based alloy stent provided by the present invention can allow the iron-based alloy substrate to undergo oxygen-consuming corrosion under the action of the degradable polymer, with minimal or no hydrogen produced, thus avoiding the clinical air embolism risk caused by a large amount of hydrogen being produced by hydrogen evolution corrosion in the prior art, and also meeting the clinical early mechanical property requirements for the stent.

Brief description of the accompanying drawings

Figure 1 is a sectional schematic diagram of a stent strut after an iron-based alloy stent provided by Example 5 is coated with a degradable polyester coating layer;

Figure 2 is a microphotograph illustrating that a small amount of hydrogen is produced in a stent strut in Example 7;

Figure 3 is a microphotograph illustrating that a small amount of hydrogen is produced in a stent strut in Example 9;

Figure 4 is a microphotograph illustrating that a small amount of hydrogen is produced in a stent strut in Example 12;

Figure 5 is a microphotograph illustrating that a small amount of hydrogen is produced in a stent strut in Example 13;

Figure 6 is a microphotograph illustrating that a small amount of hydrogen is produced in a stent strut in Example 14;

Figure 7 is a microphotograph illustrating that a large amount of hydrogen is produced in a stent strut in the corrosion process in Control Example 2.

Detailed description of the embodiments

The absorbable iron-based alloy stent is subjected to animal experiments to test and verify whether the iron-based alloy stent can rapidly and controllably corrode under the action of a degradable polymer or not, whether the iron-based alloy stent controllably corrodes or not is mainly determined by early mechanical properties and whether a large amount of hydrogen is produced at predetermined observation points in time or not, and whether the iron-based stent rapidly corrodes or not is determined by a mass loss test.

Specifically, after the iron-based alloy stent containing the degradable polymer was implanted into an animal body, the test was carried out at each of the predetermined observation points in time. For example, at 3 months from the date of implantation, an OCT follow-up test was carried out, it was found that there was no obvious difference between the surrounding area of the stent strut at this observation point and that of the original stent strut at the beginning of implanting, or animals were killed humanely, the stent and the tissue in which the stent is placed were taken out of the body, and the stent together with the blood vessel in which the stent is placed were subject to a radial support force test to determine if the stent meets the early mechanical properties; a stent was taken out at 2 years to measure the mass loss of the stent so as to observe the corrosion situation of the stent. The stent taken out of the body was axially split at each observation point in time, and the periphery of the support strut of each observed stent was observed by a microscope at the same amplification factor so as to determine whether a large amount of hydrogen is produced in the corrosion process of the stent.

The radial support force can be tested by means of a radial support force tester RXSS0-100 produced by the MSI Company; namely, the radial support force can be obtained by taking out the stent implanted into the animal body at a predetermined observation point in time together with the blood vessel and then directly testing.

The complete corrosion is characterized by a mass loss test of an animal experiment. The mass loss test is carried out by implanting an iron-based alloy stent with an iron-based alloy substrate (i.e., a bare stent excluding a degradable polymer) of which the mass is Mo into the abdominal aorta of a rabbit, cutting out the iron-based alloy stent implanted into an animal body and the tissue in which the iron-based alloy stent is placed at a predetermined observation point in time, soaking the tissue together with the stent in a solution of certain concentration (such as Imol/L of a sodium hydroxide solution) so that the tissue is digested, and then taking a stent strutout of the solution, putting the stent strut into a solution of certain concentration (such as 3% of a tartaric acid solution, and/ or an organic solution) to be ultrasonically cleaned so that corrosion products on the surface of the stent are all stripped off or dissolved in the solution, taking the residual stent strut out of the solution, drying and weighing the stent strut to obtain the mass Mt. The mass loss rate W is expressed by a percentage of the weight loss difference value of the stent strut after corrosion and cleaning in weight of the iron-based alloy substrate, as shown in Formula 1-1:

$$W = \frac{(M_0 - M_t)}{M_0} \times 100\%$$

W - Mass loss rate

Mt - Mass of the residual stent strut after corrosion

Mo - Mass of the iron-based alloy substrate

When the mass loss rate W of the stent is more than or equal to 90 percent, the iron-based alloy stent is deemed to completely corroded. The weight average molecular weight and the polydispersity index of the degradable polymer were tested by using an eight-angle laser light scattering instrument produced by the Wyatt Technology Corporation.

The absorbable iron-based alloy stent provided by the present invention is further illustrated in conjunction with the following accompanying drawings and examples. It should be understood that the following examples are only preferred embodiments.

Example 1

A pure iron stent comprises a pure iron substrate and a degradable polymer coating with which the surface of the pure iron substrate is coated, wherein the mass ratio of the pure iron substrate to the degradable polymer is 5:1. The degradable polymer is polyglycolic acid (PLA) with a weight average molecular weight of 200,000 and a polydipersity index of 1.8, and the wall thickness of the iron substrate is between 80 $\mu$m and 90 $\mu$m, and the thickness of the degradable polymer coating is between 15 $\mu$m and 20 $\mu$m. The stent was implanted into the abdominal aorta of a rabbit. The stent and the tissue in which the stent was placed were taken out at 3 months after the date of implantation, a radial support force test was carried out, and the test result that the radial support force was 70 kPa was obtained, indicating that the degradable polymer was well matched with the iron-based alloy substrate, and the early mechanical properties of the stent could be ensured; the periphery of the stent strut was observed with a microscope, no hydrogen bubbles were found. After 2 years from the date of implantation, a mass loss test was carried out by sampling again, and the mass loss rate of the stent was 95 percent, indicating that the stent completely corroded; no hydrogen bubbles were found by observing the periphery of the stent strut with a microscope.

Example 2

The surface of a bare nitrided pure iron stent (i.e., a nitrided pure iron substrate) of which the wall thickness is between 65 $\mu$m and 75 $\mu$m was uniformly coated with a 10 to 12 $\mu$m thick degradable polymer coating, wherein the mass ratio of the nitrided pure iron substrate to the degradable polymer is 25, and the degradable polymer coating is a poly(DL-lactic acid) coating with a weight average molecular weight of 100,000 and a polydispersity index of 3. The absorbable iron-based alloy stent was obtained after drying. The iron-based alloy stent was implanted into the coronary artery of a pig. At 3 months from the date of implantation, it was found that there was no difference between the surrounding area of the stent strut and the surrounding area of the stent strut at the beginning of implanting by OCT follow-up. The stent was taken out at 1 year after the implanting, the mass loss rate of the stent was 92 percent by a mass loss test, indicating that the stent completely corroded. At 3 months and 1 year after the date of implantation, the stent was taken out, and then no hydrogen bubbles were produced by observing the periphery of the stent strut with a microscope respectively.

Example 3

The surface of a bare electrodeposited pure iron (550 °C annealing) stent (i.e., an electrodeposited pure iron substrate) of which the wall thickness is between 40 $\mu$m and 50 $\mu$m was uniformly coated with a 3 to 5 $\mu$m thick mixture coating ofpolycaprolactone (PCL) and paclitaxel, wherein the mass ratio of the electrodeposited pure iron substrate to the degradable polymer was 35 : 1, the polycaprolactone (PCL) was formed by mixing two kinds ofpolycaprolactones (PCL) with weight average molecular weights of 30,000 and 80,000 according to a ratio of 1 to 2, the polydispersity index of the mixed polycaprolactones (PCL) was 25, and the mass ratio of polycaprolactones (PCL) to paclitaxel was 2 to 1. An absorbable iron-based alloy stent was obtained after drying. The iron-based alloy stent was implanted into the abdominal aorta of a rabbit. The stent was taken out at a corresponding observation point in time, the surface of the stent was observed with a microscope, and the radial support force and the mass loss percentage of the stent were tested. The test results showed that the radial support force was 60 kPa at 3 months after the date of implantation; after 1 year from the date of implantation, the mass loss rate of the stent was 98 percent, indicating that the stent completely corroded, and there were no hydrogen bubbles around the stent strut by observing with a microscope at the two observation points in time.

Example 4

The outer wall surface of a bare carburized iron stent (i.e., a carburized iron substrate) obtained after heat treatment was coated with a poly (L-lactic acid) coating, wherein the wall thickness of the carburized iron substrate is between 140 $\mu$m and 160 $\mu$m, the thickness of the poly (L-lactic acid) coating is between 30 $\mu$m and 35 $\mu$m, and the mass ratio of the carburized iron substrate to the poly (L-lactic acid) is 120. The coating comprises two layers, i.e., a PLLA coating with crystallinity of 50 percent as a bottom layer and a PLLA coating with crystallinity of 5 percent as a top layer, the weight average molecular weights of the two layers are 600,000, and the polydispersity indexes of the two layers of poly (L-lactic acid) are 1.2. The mass ratio of the degradable polymer coating with crystallinity of 50 percent to the degradable polymer coating with crystallinity of 5 percent is 1:1. An absorbable iron-based alloy stent was obtained after drying. The stent was implanted into the abdominal aorta of a rabbit. The stent was taken out at a corresponding observation point in time, the surface of the stent was observed with a microscope, and the radial support force and the weight loss percentage of the stent were tested. The test results showed that the radial support force was 45 kPa at 6 months after the implanting; after 3 years from the date of implantation, the mass loss rate of the stent was 92 percent, and there were no hydrogen bubbles around the stent strut at the above-mentioned two observation points in time.

Example 5

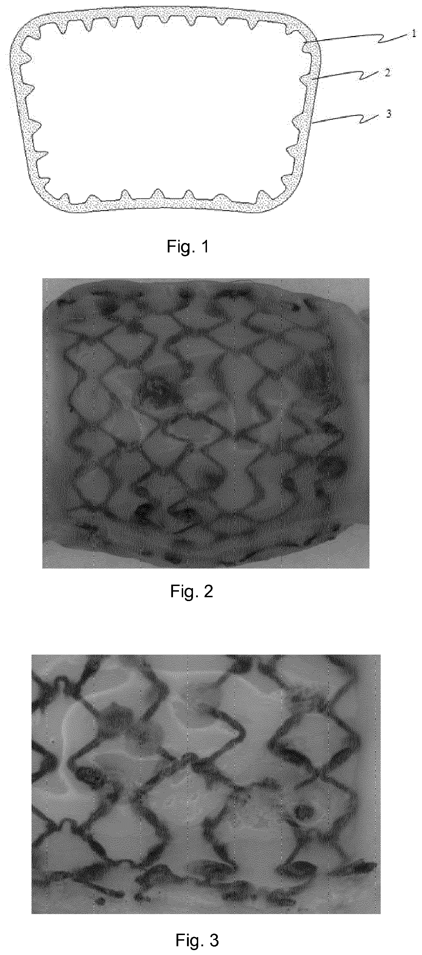

A bare iron-manganese alloy stent (i.e., an iron-manganese alloy substrate) was polished so that grooves were distributed in the surface of the stent. As shown in Figure 1, a stent strut of the stent has a thickness of between 100 $\mu$m and 120 $\mu$m, and grooves 2 are arranged in the surface of the stent strut 1. A degradable polyester polymer mixture coating 3 was uniformly coated on the surface of the stent strut 1 and in the grooves 2. The degradable polyester polymer coating was formed by mixing poly (L-lactic acid) with a weight average molecular weight of 1,000,000 and poly (lactic-glycolic acid) with a weight average molecular weight of 20,000 (the molar ratio of lactic acid to glycolic acid is 50:50) according to a mass ratio of 5 to 1, wherein the polydispersity index of the polyglycolic acid is 10 after mixing, the thickness of the mixture coating is between 20 $\mu$m and 25 $\mu$m, and the mass ratio of the iron-based alloy substrate to the degradable polymer is 40:1. An absorbable iron-based alloy stent was obtained after drying. The stent was implanted into the abdominal aorta of a pig. A stent was taken out at a corresponding observation point in time, and then the mass loss rate and the radial support force of the stent were tested. The test results showed that the radial support force was 60 kPa at 3 months after the implanting; after 2 years from the date of implantation, the mass loss rate of the stent was 95% by a mass loss test, and there were no hydrogen bubbles around the stent strut by observing at the above-mentioned two observation points in time.

Example 6

The outer surface of a bare iron-carbon alloy stent (i.e., an iron-carbon alloy substrate) with a thickness of between 30 $\mu$m and 40 $\mu$m, excluding the inner wall of a tubular cavity of the stent, was uniformly coated with a 5 to 8 $\mu$m thick poly(butylene succinate)(PBS) coating, wherein the mass ratio of the iron-carbon alloy substrate topoly(butylene succinate)(PBS) is 12:1, and the poly(butylene succinate)(PBS) has a weight average molecular weight of 60,000 and a polydispersity index of 2. An absorbable iron-based alloy stent was obtained after drying. The stent was implanted into the abdominal aorta of a rabbit. The stent was taken out at a corresponding observation point in time, and a mass loss test and a radial support force test of the stent were carried out. The results showed that the radial support force of the stent was 50 kPa at 1 month after the implanting; after 1.5 years from the date of implantation, the mass loss rate of the stent was 99 percent, and no hydrogen bubbles were found by observing the periphery of the stent strut with a microscope at the above-mentioned observation points in time.

Example 7

The surface of a bare sulfurized iron stent (i.e., a sulfurized iron-based alloy substrate) with a wall thickness of between 250 $\mu$m and 270 $\mu$m was uniformly coated with a 35 to 45 $\mu$m thick degradable polymer coating. The mass ratio of the sulfurized iron-based alloy substrate to the degradable polymer is 50:1, and the degradable polymer is formed by mixing polylactic acid (PLA) and PLGA, wherein the polylactic acid has a weight average molecular weight of 30,000, crystallinity of 40 percent, the content of 90 percent, and a polydispersity index of 1.8, and the PLGA has a weight average molecular weight of 30,000, a polydispersity index of 4, crystallinity of 5 percent and the content of 10 percent. An absorbable iron-based alloy stent was obtained after drying. The stent was implanted into the abdominal aorta of a pig. The stent was taken out at a corresponding observation point in time, and a mass loss test of the iron-based alloy stent was carried out. The test results showed that the radial support force of the stent was 50 kPa at 6 months after the date of implantation, and it was found that the periphery of few stent strut slightly bulges, i.e., a small amount of hydrogen was produced by observing the periphery of the iron-based alloy stent strut with a microscope as shown in Figure 2; after 4.5 years from the date of implantation, the mass loss rate of the stent was 90 percent, and no hydrogen bubbles were found.

Example 8

The surface of a bare iron-manganese alloy stent (i.e. an iron-manganese alloy substrate) with a wall thickness of between 120 $\mu$m and 150 $\mu$m was coated with a 15 to 20 $\mu$m thick coating. The coating was formed by mixing poly (beta-hydroxybutyrate) (PHB), poly (fumaric - sebacic acid) and heparin according to a mass ratio of 8 to 1 to 1, wherein the mass ratio of the iron-based alloy substrate to a degradable polymer, i.e., the mass sum of poly (beta-hydroxybutyrate) (PHB) and poly (fumaric - sebacic acid) is 80, PLLA has a weight average molecular weight of 300,000, crystallinity of 30%, and a polydispersity index of 2, and the PLGA has a weight average molecular weight of 100,000 and a polydispersity index of 45. An absorbable iron-based alloy stent was obtained after drying. The stent was implanted into the abdominal aorta of a rabbit. The stent was taken out at a corresponding observation point in time, and then the radial support force test and the mass loss test of the iron-based alloy stent were carried out. The results showed that that the radial support force of the iron-based alloy stent was 60 kPa at 3 months after the date of implantation; after 3 years from the date of implantation, the mass loss rate of the stent was 95 percent, and no hydrogen bubbles were found by observing the periphery of the stent strut with a microscope at the above-mentioned observation points in time.

Example 9

The surface of a bare carburized iron stent (i.e., a carburized iron substrate) with a wall thickness of between 50 $\mu$m and 70 $\mu$m was coated with a degradable polymer coating with an average thickness of between 12 $\mu$m and 15 $\mu$m. The degradable polymer coating was formed by mixing poly (DL-lactic acid) (PDLLA) and rapamycin according to a mass ratio of 2 to 1, wherein the PDLLA has a weight average molecular weight of 200,000 and a polydispersity index of 1.6, and the mass ratio of the carburized iron substrate to the degradable polymer coating is 30. An absorbable iron-based alloy stent was obtained after drying. The iron-based alloy stent was implanted into the coronary artery of a pig. The iron-based alloy stent was taken out at a corresponding observation point in time to undergo a mass loss test and a radial support force test. The results showed that at 3 months after the date of implantation, the radial support force was 60kPa, and it was found that a small amount of hydrogen was produced in the local stent strut, and few iron-based alloy stent coating slightly bulges by observing the periphery of the iron-based alloy stent strut with a microscope as shown in Figure 3. At 2 years after the date of implantation, the mass loss rate of the stent was 98 percent, and no hydrogen bubbles were found.

Example 10

The surface of a bare pure iron stent (i.e., a pure iron substrate) of which the wall thickness is between 50 $\mu$m and 60 $\mu$m was uniformly coated with a 8 to 12 $\mu$m thick degradable polymer coating, wherein the mass ratio of the pure iron substrate to the degradable polymer coating is 20:1, the degradable polymer as a bottom layer in the degradable polymer coating is PLLA with a weight average molecular weight of 300,000 and a thickness of about 6 to 8 $\mu$m, the top layer is PDLLA with a weight average molecular weight of 300,000, and the polydispersity index of the degradable polymer coating is 15. An iron-based alloy stent was obtained after drying. The iron-based alloy stent was implanted into the coronary artery of a pig. OCT follow-up was carried out at 3 months after the date of implantation, the OCT testing results showed that there was no difference between the surrounding area of the iron-based alloy stent and the surrounding area of the iron-based alloy stent at the beginning of implanting. After 2.5 years from the date of implantation, a mass loss test of the stent was carried out by sampling, the mass loss rate of the stent was 98 percent, and it was found that no hydrogen bubbles were produced by observing the periphery of a stent strut by sampling at the above-mentioned two observation points in time.

Example 11

The surface of a bare nitrided iron stent (i.e. a nitrided iron substrate) of which the wall thickness is between 60 $\mu$m and 90 $\mu$m was coated with a 10 to 15 $\mu$m thick poly(3-hydroxybutyrate-co-3-hydroxyvalerate) (PHBV) coating with a weight average molecular weight of 400,000 and a polydispersity index of 3, and then coated with a mixed coating comprising polylactic acid (PLA), poly(erucic acid dimer-sebacic acid) and cilostazol by spraying again. The mixed coating with a thickness of about 10 $\mu$m was mainly sprayed on the outer wall and the side walls of the nitrided iron substrate, wherein the polylactic acid (PLA) has a weight average molecular weight of 50,000 and a polydispersity index of 1.6, the poly (erucic acid dimer- sebacic acid) has a weight average molecular weight of 20,000 and a polydispersity index of 10, the mass ratio of the polylactic acid (PLA), the poly (erucic acid dimer- sebacic acid) to the cilostazol is 1:1:1, and the ratio of the mass of the nitrided iron substrate to the mass sum of the two degradable polymer coatings is 35:1. An iron-based alloy stent was obtained after drying. The iron-based alloy stent was implanted into the coronary artery of a pig. OCT follow-up was carried out at 3 months after the date of implantation, and the OCT testing results showed that there was no difference between the tubular cavity area of the iron-based alloy stent and the tubular cavity area of the iron-based alloy stent at the beginning of implanting. After 1.5 years from the date of implantation, the mass loss rate of the stent was 95 percent, and it was found that no hydrogen bubbles were produced around the stent strut by sampling at the two points in time.

Example 12

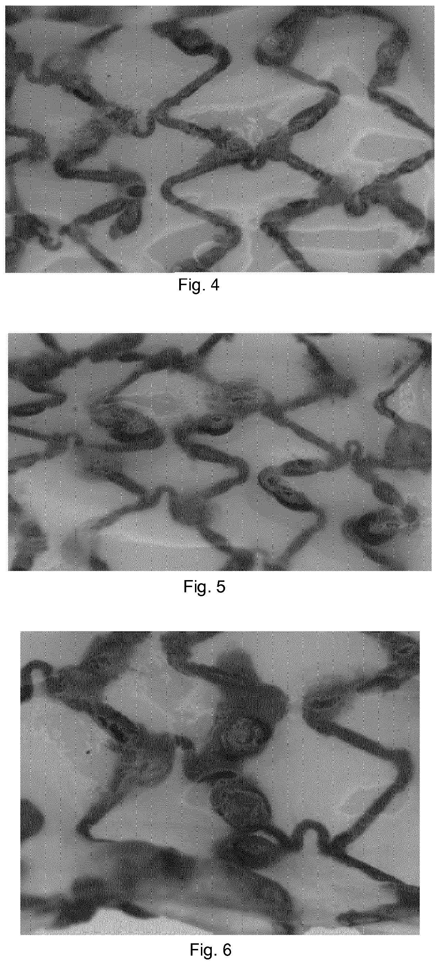

A stent strut of a bare nitrided iron stent (i.e., a nitrided iron substrate) of which the wall thickness is between 220 μm and 240 μm was treated so that micropores and grooves are arranged in the stent strut, and poly (butylene succinate)(PBS) is uniformly filled in the micropores and the grooves, wherein the poly (butylene succinate)(PBS) has a weight average molecular weight of 150,000 and a polydispersity index of 5, and the mass ratio of the nitrided iron substrate to the poly (butylene succinate)(PBS) is 5:1. An iron-based alloy stent was obtained after drying. The stent was implanted into a rabbit body. The iron-based alloy stent was taken out at a corresponding observation point in time, and a mass loss test and a radial support force test were carried out. At 2 months after the date of implantation, the radial support force of the stent was 75 kPa, and it was found that a small amount of bubbles was produced by observing the periphery of the stent strut with a microscope shown in Figure 4. The mass loss rate of the iron-based alloy stent was 90 percent and no hydrogen bubbles were found around the stent strut at 3 years after the date of implantation.

Example 13

An iron-cobalt alloy stent comprises an iron-cobalt alloy substrate and a degradable polymer coating covering the surface of the substrate, wherein the wall thickness of the iron-cobalt alloy substrate is in the range of 280 μm to 300 μm, the degradable polymer coating is a copolymer coating formed by copolymerizing monomers forming PLLA and PGA, the mass ratio of the monomers forming the two kinds of degradable polymers is 9:1, the copolymer has a weight average molecular weight of 50,000, a polydispersity index of 1.1 and crystallinity of 50 percent, and the thickness of the copolymer coating is in the range of 35 μm to 45 μm. The copolymer coating also comprises rapamycin, the ratio of the mass sum of the two kinds of polymers to the mass of the drug is 0.1:1, and the mass ratio of the iron-cobalt alloy substrate to the polymer coating is 25:1. The iron-cobalt alloy stent was implanted into the abdominal aorta of a pig. The radial support force was tested by sampling, and the periphery of the stent strut was observed with a microscope at 3 months and 4.5 years after the date of implantation. The test results showed that at 3 months after the date of implantation, the radial support force of the iron-based alloy stent was 45 kPa and a small amount of hydrogen bubbles was produced around the stent strut as shown in Figure 5; the mass loss rate of the stent strut was 90 percent, and no hydrogen bubbles were produced around the stent strut at 5 years after the date of implantation.

Example 14

The surface of a bare iron-carbon alloy stent (i.e., an iron-carbon alloy substrate) was coated with a degradable polyester coating, wherein the wall thickness of the iron-carbon alloy substrate is in the range of 180 μm to 200 μm, the thickness of the degradable polyester coating is in the range of 20 μm to 25 μm. The degradable polyester coating is formed by mixing poly (butylene succinate) (PBS) and polyglycolic acid (PGA) according to a mass ratio of 9:1, and the blend has a weight average molecular weight of 250,000 and a polydispersity index of 2. The degradable polyester coating may also be mixed with heparin, wherein the mass ratio of the degradable polyester to the heparin is 20:1, and the mass ratio of the iron-carbon alloy substrate to the degradable polyester is 40:1. An iron-based alloy stent was obtained after drying. The iron-based alloy stent was implanted into the abdominal aorta of a pig. The radial support force was tested by sampling, and the periphery of a stent strut was observed with a microscope at 3 months and 3 years after the date of implantation. The test results showed that the radial support force was 75 kPa at 3 months after the date of implantation, and a small amount of hydrogen bubbles was produced around the stent strut as shown in Figure 6; the mass loss rate of the stent strut was 95 percent, and no hydrogen bubbles were produced around the stent strut at 3 years after the date of implantation.

Example 15

An iron-nitrogen alloy stent comprises an iron-nitrogen alloy substrate and a degradable polymer coating covering the surface of the substrate, wherein the wall thickness of the iron-nitrogen alloy substrate is in the range of 90 μm to 100 μm, and the thickness of the degradable polymer coating is in the range of 15 μm to 20 μm. The coating is formed by mixing polylactic acid (PLA) and poly(ethyleneglycol adipate) (PEA) according to a mass ratio of 1 to 5, wherein the weight average molecular weights of the polylactic acid (PLA)and the poly(ethyleneglycol adipate) (PEA) are 500,000 and 300,000 respectively, the polydispersity index of the degradable polyester coating is 3, and the mass ratio of the iron-nitrogen alloy substrate to the degradable polyester coating is 10:1. The iron-based alloy stent was implanted into the abdominal aorta of a rabbit. The radial support force was tested by sampling, and the periphery of a stent strut was observed by a microscope at 3 months and 3 years after the date of implantation. The test results showed that the radial support force of the iron-based alloy stent was 50 kPa, and no hydrogen bubbles were produced around the stent strut at 3 months after the date of implantation; the mass loss rate of the stent strut was 95 percent, and no hydrogen bubbles were produced around the stent strut at 3 years after the date of implantation.

Example 16

An iron-palladium alloy stent comprises an iron-palladium alloy substrate and a degradable polymer coating covering the surface of the substrate, wherein the wall thickness of the iron-palladium alloy substrate is in the range of 70 $\mu$m to 90 $\mu$m, and the thickness of the degradable polyester coating is in the range of 10 $\mu$m to 15 $\mu$m. The degradable polyester coating is formed by mixing polylactic acid (PLA) and polyglycolic acid (PGA) according to a mass ratio of 5 to 1, wherein the weight average molecular weights of the polylactic acid (PLA) and the polyglycolic acid (PGA) are 800,000 and 20,000 respectively, the polydispersity index of the mixture is 50, and the mass ratio of the iron-palladium alloy substrate to the degradable polyester coating is 15:1. The iron-based alloy stent was implanted into the abdominal aorta of a rabbit. The radial support force was tested by sampling, and the periphery of a stent strut was observed with a microscope at 2 months and 2 years after the date of implantation. The test results showed that the radial support force of the stent was 80 kPa, and no hydrogen bubbles were produced around the stent strut at 2 months after the date of implantation; the mass loss rate of the stent was 98 percent, and no hydrogen bubbles were produced around the stent strut at 2 years after the date of implantation.

There were thickness differences between every part of each absorbable iron-based alloy stent in the preparation process, therefore, the coating thickness and the wall thickness of the iron-based alloy substrate were interval values in the Examples 1 to 16, and whether hydrogen bubbles were produced around the stent or not at predetermined observation points in time was observed with a microscope at the same magnification factor in each Example.

Control example 1

A bare pure iron stent (a pure iron substrate, uncoated with any coating on the surface) of which the wall thickness is between 60 $\mu$m and 70 $\mu$m was implanted into the abdominal aorta of a rabbit. After 3 months from the date of implantation, the stent was taken out, and the radial support force was 120 kPa by testing; at 3 years after the date of implantation, the stent was taken out to undergo a mass loss test, and the mass loss rate of the stent was 25 percent at the moment, indicating that the bare pure iron stent corroded slowly.

Control example 2

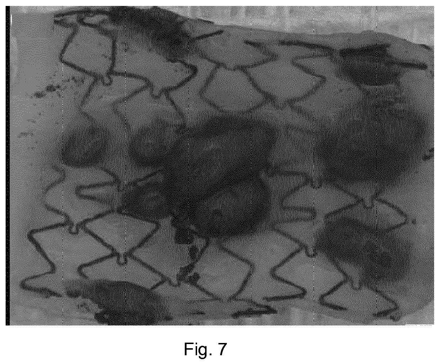

The surface of a bare pure iron stent (i.e., a pure iron substrate) of which the wall thickness is in the range of 60 $\mu$m to 70 $\mu$m was coated with a 25 $\mu$m to 35$\mu$m thick polylactic acid (PLA) coating, wherein the mass ratio of the pure iron substrate to the polylactic acid (PLA) is 10: 1, and the polylactic acid (PLA) has a weight average molecular weight of 15,000 and a polydispersity index of 1.8. An iron-based stent was obtained after drying. The iron-based stent was implanted into the abdominal aorta of a rabbit. After 1 month from the date of implantation, the stent was taken out and axially split, and the periphery of the support strut was observed with a microscope at the same amplification factor as those of the above-mentioned Examples; it was found that a large amount of hydrogen was produced around the stent strut in the corrosion process, and larger hydrogen bulges were formed as shown in Figure 7, indicating that there was a relatively large risk of air embolism formation. The radial support force was 20 kPa by testing at 3 months after the date of implantation, and the mass loss test of the stent showed that the mass loss rate of the stent was 100 percent at 6 months after the date of implantation, indicating that the stent completely corroded, the corrosion was too rapid, and the clinical mechanical property requirement could not be met at predetermined points in time.

It can be seen from the test results of the above-mentioned Examples 1 to 16 and Control examples 1 to 2 that the absorbable iron-based alloy stent provided by the present invention uses the degradable polymer with a weight average molecular weight in the range of 20,000 to 1,000,000 and a polydispersity index of more than 1.0 and less than or equal to 50 to achieve a result that no hydrogen is produced or only a small amount of hydrogen is produced in 5 years after the iron-based alloy substrate is implanted into the body, i.e., the oxygen-consuming corrosion mainly occurs, and the internal corrosion rate is improved by means of oxygen-consuming corrosion, thereby overcoming the technical biases that the corrosion rate of an iron-based alloy can only be improved by hydrogen evolution corrosion in the degradation of the iron-based alloy and that the degradation speed of the iron-based alloy is is not easily improved by increasing the oxygen-consuming corrosion rate in the prior art, and further avoiding the clinical air embolism risk brought by a large amount of hydrogen produced from the iron-based alloy substrate due to hydrogen evolution corrosion in the prior art. In addition, the mass loss rate of the absorbable iron-based alloy stent provided by the invention is not less than 90 percent in 5 years after the implantation into the body, thus meeting the clinical corrosion period requirements for the degradable stent; when the OCT follow-up was carried out, there was no obvious difference between the surrounding area of the stent and the surrounding area of the stent at the beginning of implanting, or the early radial support forces were all more than 23.3 kPa (175 mm mercury column), thus meeting the clinical early mechanical property requirements for the stent after the implantation into the body.

Claims

1. An absorbable iron-based alloy stent, comprising an iron-based alloy substrate and a degradable polyester in contact with the surface of the substrate, wherein the degradable polyester has a weight average molecular weight in the range of 20,000 to 1,000,000 and a polydispersity index more than or equal to 1.2 and less than or equal to 30, or more than 1.0 and less than 1.2, or more than 30 and less than or equal to 50, and wherein the surface of the iron-based alloy substrate is coated with degradable polyester in the form of a coating layer, **characterized in that** when a wall thickness of the iron-based alloy substrate is more than or equal to 30 $\mu$m and less than 50 $\mu$m, a thickness of the degradable polyester coating layer is more than or equal to 3 $\mu$m and less than 5 $\mu$m, or more than or equal to 5 $\mu$m and less than 10 $\mu$m, or more than or equal to 10 $\mu$m and less than 15 $\mu$m, or more than or equal to 15 $\mu$m and less than or equal to 20 $\mu$m; or when the wall thickness of the iron-based alloy substrate is more than or equal to 50 $\mu$m and less than 100 $\mu$m, the thickness of the degradable polyester coating layer is more than or equal to 5 $\mu$m and less than 10 $\mu$m, or more than or equal to 10 $\mu$m and less than 15 $\mu$m, or more than or equal to 15 $\mu$m and less than 20 $\mu$m, or more than or equal to 20 $\mu$m and less than or equal to 25 $\mu$m; or when the wall thickness of the iron-based alloy substrate is more than or equal to 100 $\mu$m and less than 200 $\mu$m, the thickness of the degradable polyester coating layer is more than or equal to 10 $\mu$m and less than 15 $\mu$m, or more than or equal to 15 $\mu$m and less than 20 $\mu$m, or more than or equal to 20 $\mu$m and less than 25 $\mu$m, or more than or equal to 25 $\mu$m and less than or equal to 35 $\mu$m; or when the wall thickness of the iron-based alloy substrate is more than or equal to 200 $\mu$m and less than or equal to 300 $\mu$m, and the thickness of the degradable polyester coating layer is more than or equal to 10 $\mu$m and less than 15 $\mu$m, or more than or equal to 15 $\mu$m and less than 20 $\mu$m, or more than or equal to 20 $\mu$m and less than 25 $\mu$m, or more than or equal to 25 $\mu$m or less than 35 $\mu$m, or more than or equal to 35 $\mu$m and less than or equal to 45 $\mu$m.

2.  The absorbable iron-based alloy stent as set forth in claim 1, **characterized in that** the weight average molecular weight of the degradable polyester is more than or equal to 20,000 and less than 100,000, or more than or equal to 100,000 and less than 250,000, or more than or equal to 250,000 and less than 400,000, or more than or equal to 400,000 and less than 600,000, or more than or equal to 600,000 and less than or equal to 1,000,000.

3.  The absorbable iron-based alloy stent as set forth in claim 1, **characterized in that** the polydispersity index is more than or equal to 1.2 and less than 2, or more than or equal to 2 and less than 3, or more than or equal to 3 and less than 5, or more than or equal to 5 and less than 10, or more than or equal to 10 and less than 20, or more than or equal to 20 and less than or equal to 30.

4.  The absorbable iron-based alloy stent as set forth in claim 1, **characterized in that** the mass ratio of the iron-based alloy substrate to the degradable polyester is more than or equal to 1 and less than or equal to 200.

5.  The absorbable iron-based alloy stent as set forth in claim 4, **characterized in that** the mass ratio of the iron-based alloy substrate to the degradable polyester is more than or equal to 5 and less than or equal to 50.

6.  The absorbable iron-based alloy stent as set forth in claim 1, **characterized in that** the iron-based alloy substrate is provided with gaps or grooves, and the degradable polyester is arranged in the gaps or grooves; or/and the iron-based alloy substrate is provided with an inner cavity, and the degradable polyester is filled in the inner cavity.

7.  The absorbable iron-based alloy stent as set forth in claim 1, **characterized in that** the degradable polyester is any one of the following: polylactic acid, polyglycolic acid, poly(butylene succinate}, poly (beta-hydroxybutyrate}, polycaprolactone, poly(ethyleneglycol adipate), poly(lactic-co-glycolic acid}, and poly(3-hydroxybutyrate-co-3- hydroxyvalerate); or
the degradable polyester comprises at least two kinds of degradable polyester polymers with high molecular weights, wherein each of the two kinds of degradable polyester polymers with high molecular weight has a weight average molecular weight of more than or equal to 100,000 and less than 200,000, or more than or equal to 200,000 and less than 400,000, or more than or equal to 400,000 and less than 600,000, or more than or equal to 600,000 and less than or equal to 1000,000; or
the degradable polyester is a mixture of at least two of polylactic acid, polyglycolic acid, poly(butylene succinate), poly (beta-hydroxybutyrate), polycaprolactone, poly(ethyleneglycol adipate}, poly(lactic-co-glycolic acid), and poly(3-hydroxybutyrate-co-3-hydroxyvalerate), or a copolymer formed by copolymerizing at least two of monomers forming polylactic acid, polyglycolic acid, poly(butylene succinate}, poly (beta-hydroxybutyrate}, polycaprolactone, poly(ethyleneglycol adipate}, poly(lactic -co-glycolic acid), and poly(3-hydroxybutyrate-co-3-hydroxyvalerate); or
the degradable polyester is a mixture of at least two kinds of degradable polyester polymers with different crystallinity, wherein the content of degradable polyester polymer with crystallinity more than or equal to 5% and less than or equal to 50% is more than or equal to 10% and less than or equal to 90% in percentage by mass, and the degradable polyester polymer is selected from the group consisting of polylactic acid, polyglycolic acid, poly (butylene succinate), poly (beta-hydroxybutyrate), polycaprolactone, poly (ethyleneglycol adipate), poly(lactic- co -glycolic acid), and poly (3-hydroxybutyrate-co- 3-hydroxyvalerate).

8. The absorbable iron-based alloy stent as set forth in claim 1, **characterized in that** the degradable polyester comprises at least two kinds of the same type of degradable polyester polymers, wherein the first kind of degradable polyester polymer has a weight average molecular weight of more than or equal to 20,000 and less than 100,000, the second kind of degradable polyester polymer has a weight average molecular weight more than or equal to 100,000 and less than or equal to 1,000,000, the mass ratio of the first kind of degradable polyester polymer to the second kind of degradable polyester polymer is in the range of 1:9 to 9:1 and optionally in the range of 1:5 to 5:1, and the same type of degradable polyester polymer is any one of the followings: polylactic acid, polyglycolic acid, poly(butylene succinate), poly (beta-hydroxybutyrate), polycaprolactone, poly(ethyleneglycol adipate), poly(lactic-co-glycolic acid), and poly(3-hydroxybutyrate-co-3-hydroxyvalerate).

9. The absorbable iron-based alloy stent as set forth in claim 1, **characterized in that** the degradable polyester is mixed with an active drug, and the mass ratio of the degradable polyester to the drug is in the range of 0.1 to 20, optionally in the range 0.5 to 10.

10. The absorbable iron-based alloy stent as set forth in claim 1, **characterized in that** the iron-based alloy substrate is selected from pure iron or an iron-based alloy formed by doping at least one of C, N, O, S, P, Mn, Pd, Si, W, Ti, Co, Cr, Cu, and Re into pure iron.

11. An absorbable iron-based alloy stent, comprising an iron-based alloy substrate and a degradable polymer in contact with the surface of the substrate, wherein the degradable polymer has a weight average molecular weight more than or equal to 20,000 and less than or equal to 1,000,000 and a polydispersity index of more than 1.0 and less than or equal to 50, and the degradable polymer is capable of producing a carboxyl group after the iron-based alloy stent is implanted into the body, and wherein the surface of the iron-based alloy substrate is coated with the degradable polymer in the form of a coating layer, **characterized in that** when a wall thickness of the iron-based alloy substrate is more than or equal to 30 $\mu$m and less than 50 $\mu$m, a thickness of the degradable polymer coating layer is more than or equal to 3 $\mu$m and less than 5 $\mu$m, or more than or equal to 5 $\mu$m and less than 10 $\mu$m, or more than or equal to 10 $\mu$m and less than 15 $\mu$m, or more than or equal to 15 $\mu$m and less than or equal to 20 $\mu$m; or when the wall thickness of the iron-based alloy substrate is more than or equal to 50 $\mu$m and less than 100 $\mu$m, the thickness of the degradable polymer coating layer is more than or equal to 5 $\mu$m and less than 10 $\mu$m, or more than or equal to 10 $\mu$m and less than 15 $\mu$m, or more than or equal to15 $\mu$m and less than 20 $\mu$m, or more than or equal to 20 $\mu$m and less than or equal to 25 $\mu$m; or when the wall thickness of the iron-based alloy substrate is more than or equal to 100 $\mu$m and less than 200 $\mu$m, the thickness of the degradable polymer coating layer is more than or equal to 10 $\mu$m and less than 15 $\mu$m, or more than or equal to 15 $\mu$m and less than 20 $\mu$m, or more than or equal to 20 $\mu$m and less than 25 $\mu$m, or more than or equal to 25 $\mu$m and less than or equal to 35 $\mu$m; or when the wall thickness of the iron-based alloy substrate is more than or equal to 200 $\mu$m and less than or equal to 300 $\mu$m, the thickness of the degradable polymer coating layer is more than or equal to 10 $\mu$m and less than 15 $\mu$m, or more than or equal to 15 $\mu$m and less than 20 $\mu$m, or more than or equal to 20 $\mu$m and less than 25 $\mu$m, or more than or equal to 25 $\mu$m and less than 35 $\mu$m, or more than or equal to 35 $\mu$m and less than or equal to 45$\mu$m.

12. The absorbable iron-based alloy stent as set forth in claim 11, **characterized in that** the degradable polymer is a degradable polyester; or the degradable polymer is a blend of the degradable polyester and a degradable polyanhydride; or the degradable polymer is a degradable copolymer formed by copolymerizing monomers forming the degradable polyester and the degradable polyanhydride, and the degradable polyester and the degradable polyanhydride have a weight average molecular weight more than or equal to 20,000 and less than or equal to 1,000,000, and polydispersity indexes of more than 1.0 and less than or equal to 50.

13. The absorbable iron-based alloy stent as set forth in claim 12, **characterized in that** the polydispersity indexes of the degradable polyester and the degradable polyanhydride are more than or equal to 1.2 and less than 2, or more than or equal to 2 and less than 3, or more than or equal to 3 and less than 5, or more than or equal to 5 and less than 10, or more than or equal to 10 and less than 20, or more than or equal to 20 and less than 30, or more than or equal to 30and less than or equal to 50.

14. The absorbable iron-based alloy stent as set forth in claim 12, **characterized in that** the mass ratio of the iron-based alloy substrate to the degradable polymer is in the range of 1 to 200, optionally in the range of 5 to 50.

15. The absorbable iron-based alloy stent as set forth in claim 12, **characterized in that** the weight average molecular weight of the degradable polyester is more than or equal to 20,000 and less than 100,000, or more than or equal to 100,000 and less than 250,000, or more than or equal to 250,000 and less than 400,000, or more than or equal to 400,000 and less than 600,000, or more than or equal to 600,000 and less than 1,000,000.

16. The absorbable iron-based alloy stent as set forth in claim 11, **characterized in that** the iron-based alloy substrate is provided with gaps or grooves, and the degradable polymer is arranged in the gaps or grooves; or/and the iron-based alloy substrate is provided with an inner cavity, and the degradable polymer is filled in the inner cavity.

17. The absorbable iron-based alloy stent as set forth in claim 12, **characterized in that** the degradable polyester is any one of the followings: polylactic acid, polyglycolic acid, poly(butylene succinate), poly (beta-hydroxybutyrate), polycaprolactone, poly(ethyleneglycol adipate), poly(lactic-co-glycolic acid), and poly(3-hydroxybutyrate-co-3- hydroxyvalerate); or the degradable polyester comprises at least two kinds of the same type of degradable polyester polymers, wherein the first kind of degradable polyester polymer has a weight average molecular weight of more than or equal to 20,000 and less than 100,000, the second kind of degradable polyester polymer has a weight average molecular weight more than or equal to 100,000 and less than or equal to 1,000,000, the mass ratio of the first kind of degradable polyester polymer to the second kind of degradable polyester polymer is more than or equal to 1:9 and less than or equal to 9:1, and the same type of degradable polyester polymer is any one of the followings: polylactic acid, polyglycolic acid,poly(butylene succinate), poly (beta-hydroxybutyrate), polycaprolactone, poly(ethyleneglycol adipate), poly(lactic-co-glycolic acid) and poly(3-hydroxybutyrate-co-3-hydroxyvalerate); or the degradable polyester comprises at least two kinds of degradable polyester polymers with high molecular weights, and each of the at least two kinds of degradable polyester polymers with high molecular weights has a weight average molecular weight of more than or equal to 100,000 and less than 200,000, or more than or equal to 200,000 and less than 400,000, or more than or equal to 400,000 and less than 600,000, or more than or equal to 600,000 and less than or equal to 1,000,000; or the degradable polyester is a blend of at least two of polylactic acid, polyglycolic acid, poly(butylenesuccinate), poly (beta-hydroxybutyrate), polycaprolactone, poly(ethyleneglycol adipate), poly(lactic-co-glycolic acid), and poly(3-hydroxybutyrate-co-3-hydroxyvalerate), or a copolymer formed by copolymerizing at least two of monomers forming polylactic acid, polyglycolic acid, poly(butylene succinate), poly (beta-hydroxybutyrate), polycaprolactone, poly(ethyleneglycol adipate), poly(lactic-co-glycolic acid), and poly(3-hydroxybutyrate-co-3-hydroxyvalerate); or the degradable polyester is a mixture of at least two kinds of degradable polyester polymers with different crystallinity, wherein the content of degradable polyester polymer with crystallinity in the range of 5% to 50% is in the range of 10% to 90% in percentage by mass, and the degradable polyester polymer is selected from the group consisting of polylactic acid, polyglycolic acid, poly (butylene succinate), poly (beta-hydroxybutyrate), polycaprolactone, poly (ethyleneglycol adipate), poly (lactic- co -glycolic acid), and poly (3-hydroxybutyrate-co- 3-hydroxyvalerate).

18. The absorbable iron-based alloy stent as set forth in claim 17, **characterized in that** when the degradable polyester comprises at least two kinds of the same type of degradable polyester polymers, the mass ratio of the first kind of degradable polyester polymer to the second kind of degradable polyester polymer is in the range of 1:5 to 5:1.

19. The absorbable iron-based alloy stent as set forth in claim 12, **characterized in that** the polyanhydride is any one of the following: poly(l, 3-bis(p-carboxyphenoxy) propane-sebacic acid) and poly(erucic acid dimer-sebacic acid) or poly(fumaric - sebacic acid), the degradable polyester is any one of the followings: polylactic acid, polyglycolic acid, poly(butylene succinate), poly (beta- hydroxybutyrate), polycaprolactone, poly(ethyleneglycol adipate), poly(lactic-co-glycolic acid), and poly(3- hydroxybutyrate-co-3-hydroxyvalerate), and the mass ratio of the degradable polyester to the polyanhydride is in the range of 1:9 to 9:1.

20. The absorbable iron-based alloy stent as set forth in claim 12, **characterized in that** in the blend of the degradable polyester and the degradable polyanhydride, the content of degradable polyester or degradable polyanhydride with crystallinity in the range of 5% to 50% is in the range of 10% to 90% in percentage by mass, the degradable polyester polymer is selected from the group consisting of polylactic acid, polyglycolic acid, poly (butylene succinate), poly (beta-hydroxybutyrate), polycaprolactone, poly (ethyleneglycol adipate), poly (lactic- co-glycolic acid), and poly (3-hydroxybutyrate-co-3-hydroxyvalerate), and the polyanhydride is selected from the group consisting of poly(l, 3- bis(p-carboxyphenoxy) propane-sebacic acid) and poly(erucic acid dimer- sebacic acid) or poly(fumaric - sebacic acid).

**21.** The absorbable iron-based alloy stent as set forth in claim 11, **characterized in that** the degradable polymer is mixed with an active drug, and the mass ratio of the degradable polymer to the drug is in the range of 0.1 to 20, optionally in the range of 0.5 to 10.

**22.** The absorbable iron-based alloy stent as set forth in claim 11, **characterized in that** the iron-based alloy substrate is selected from pure iron or an iron-based alloy formed by doping at least one of C, N, 0, S, P, Mn, Pd, Si, W, Ti, Co, Cr, Cu, and Re into pure iron.

**Patentansprüche**

**1.** Resorbierbarer Stent aus einer Legierung auf Eisenbasis, mit einem Substrat aus einer Legierung auf Eisenbasis und einem abbaubaren Polyester in Kontakt mit der Oberfläche des Substrats, wobei der abbaubare Polyester eine massegemittelte Molekülmasse im Bereich von 20.000 bis 1.000.000 und einen Polydispersitätsindex von mehr als oder gleich 1,2 und weniger als oder gleich 30 oder von mehr als 1,0 und weniger als 1,2 oder von mehr als 30 und weniger als oder gleich 50 aufweist, und wobei die Oberfläche des Substrats aus der Legierung auf Eisenbasis mit abbaubarem Polyester in Form einer Beschichtungslage beschichtet ist,
**dadurch gekennzeichnet, dass**
dann, wenn eine Wandstärke des Substrats aus der Legierung auf Eisenbasis mehr als oder gleich 30 μm und weniger als 50 μm beträgt, eine Dicke der Beschichtungslage aus abbaubarem Polyester mehr als oder gleich 3 μm und weniger als 5 μm oder mehr als oder gleich 5 μm und weniger als 10 μm oder mehr als oder gleich 10 μm und weniger als 15 μm oder mehr als oder gleich 15 μm und weniger als oder gleich 20 μm beträgt;
oder dann, wenn die Wandstärke des Substrats aus der Legierung auf Eisenbasis mehr als oder gleich 50 μm und weniger als 100 μm beträgt, die Dicke der Beschichtungslage aus abbaubarem Polyester mehr als oder gleich 5 μm und weniger als 10 μm oder mehr als oder gleich 10 μm und weniger als 15 μm oder mehr als oder gleich 15 μm und weniger als 20 μm oder mehr als oder gleich 20 μm und weniger als oder gleich 25 μm beträgt;
oder dann, wenn die Wandstärke des Substrats aus der Legierung auf Eisenbasis mehr als oder gleich 100 μm und weniger als 200 μm beträgt, die Dicke der Beschichtungslage aus abbaubarem Polyester mehr als oder gleich 10 μm und weniger als 15 μm oder mehr als oder gleich 15 μm und weniger als 20 μm oder mehr als oder gleich 20 μm und weniger als 25 μm oder mehr als oder gleich 25 μm und weniger als oder gleich 35 μm beträgt;
oder dann, wenn die Wandstärke des Substrats aus der Legierung auf Eisenbasis mehr als oder gleich 200 μm und weniger als oder gleich 300 μm beträgt, die Dicke der Beschichtungslage aus abbaubarem Polyester mehr als oder gleich 10 μm und weniger als 15 μm oder mehr als oder gleich 15 μm und weniger als 20 μm oder mehr als oder gleich 20 μm und weniger als 25 μm oder mehr als oder gleich 25 μm oder weniger als 35 μm oder mehr als oder gleich 35 μm und weniger als oder gleich 45 μm beträgt.

**2.** Resorbierbarer Stent aus einer Legierung auf Eisenbasis nach Anspruch 1, **dadurch gekennzeichnet, dass** die massegemittelte Molekülmasse des abbaubaren Polyesters mehr als oder gleich 20.000 und weniger als 100.000 oder mehr als oder gleich 100.000 und weniger als 250.000 oder mehr als oder gleich 250.000 und weniger als 400.000 oder mehr als oder gleich 400.000 und weniger als 600.000 oder mehr als oder gleich 600.000 und weniger als oder gleich 1.000.000 beträgt.

**3.** Resorbierbarer Stent aus einer Legierung auf Eisenbasis nach Anspruch 1, **dadurch gekennzeichnet, dass** der Polydispersitätsindex mehr als oder gleich 1,2 und weniger als 2 oder mehr als oder gleich 2 und weniger als 3 oder mehr als oder gleich 3 und weniger als 5 oder mehr als oder gleich 5 und weniger als 10 oder mehr als oder gleich 10 und weniger als 20 oder mehr als oder gleich 20 und weniger als oder gleich 30 beträgt.

**4.** Resorbierbarer Stent aus einer Legierung auf Eisenbasis nach Anspruch 1, **dadurch gekennzeichnet, dass** das Massenverhältnis des Substrats aus der Legierung auf Eisenbasis zu dem abbaubaren Polyester mehr als oder gleich 1 und weniger als oder gleich 200 beträgt.

**5.** Resorbierbarer Stent aus einer Legierung auf Eisenbasis nach Anspruch 4, **dadurch gekennzeichnet, dass** das Massenverhältnis des Substrats aus der Legierung auf Eisenbasis zu dem abbaubaren Polyester mehr als oder gleich 5 und weniger als oder gleich 50 beträgt.

**6.** Resorbierbarer Stent aus einer Legierung auf Eisenbasis nach Anspruch 1, **dadurch gekennzeichnet, dass** das Substrat aus der Legierung auf Eisenbasis mit Spalten oder Rillen versehen ist und der abbaubare Polyester in den Spalten oder Rillen angeordnet ist,
oder/und das Substrat aus der Legierung auf Eisenbasis mit einem Innenhohlraum versehen ist und der abbaubare Polyester in den Innenhohlraum gefüllt ist.

**7.** Resorbierbarer Stent aus einer Legierung auf Eisenbasis nach Anspruch 1, **dadurch gekennzeichnet, dass** der abbaubare Polyester einer der folgenden ist: Polymilchsäure, Polyglykolsäure, Poly(butylensuccinat}, Poly(beta-hydroxybutyrat}, Polycaprolacton, Poly(ethylenglykoladipat), Poly(milch-co-glykolsäure} und Poly(3-hydroxybutyrat-co-3-hydroxyvalerat); oder

der abbaubare Polyester mindestens zwei Arten von abbaubaren Polyesterpolymeren mit hoher relativer Molekülmasse umfasst, wobei jede der beiden Arten von abbaubaren Polyesterpolymeren mit hoher relativer Molekülmasse eine massegemittelte Molekülmasse von mehr als oder gleich 100.000 und weniger als 200.000 oder mehr als oder gleich 200.000 und weniger als 400.000 oder mehr als oder gleich 400.000 und weniger als 600.000 oder mehr als oder gleich 600.000 und weniger als oder gleich 1.000.000 hat; oder

der abbaubare Polyester eine Mischung aus mindestens zweien der folgenden ist: Polymilchsäure, Polyglykolsäure, Poly(butylensuccinat), Poly(beta-hydroxybutyrat), Polycaprolacton, Poly(ethylenglykoladipat}, Poly(milch-co-glykolsäure) und Poly(3-hydroxybutyrat-co-3-hydroxyvalerat), oder ein Copolymer ist, das durch Copolymerisation von mindestens zweien aus Monomeren, die Polymilchsäure, Polyglykolsäure, Poly(butylensuccinat}, Poly(beta-hydroxybutyrat}, Polycaprolacton, Poly(ethylenglykoladipat}, Poly(milch-co-glykolsäure) und Poly(3-hydroxybutyrat-co-3-hydroxyvalerat) bilden, gebildet ist; oder

der abbaubare Polyester eine Mischung aus mindestens zwei Arten von abbaubaren Polyesterpolymeren mit unterschiedlicher Kristallinität ist, wobei der Gehalt an abbaubarem Polyesterpolymer mit einer Kristallinität von mehr als oder gleich 5% und weniger als oder gleich 50% mehr als oder gleich 10% und weniger als oder gleich 90% in Massenprozent beträgt und das abbaubare Polyesterpolymer aus der aus Polymilchsäure, Polyglykolsäure, Poly(butylensuccinat), Poly(beta-hydroxybutyrat), Polycaprolacton, Poly(ethylenglykoladipat), Poly(milch-co-glykolsäure) und Poly(3-hydroxybutyrat-co-3-hydroxyvalerat) bestehenden Gruppe ausgewählt ist.

8. Resorbierbarer Stent aus einer Legierung auf Eisenbasis nach Anspruch 1, **dadurch gekennzeichnet, dass** der abbaubare Polyester mindestens zwei Arten des gleichen Typs von abbaubaren Polyesterpolymeren umfasst, wobei die erste Art von abbaubarem Polyesterpolymer eine massegemittelte Molekülmasse von mehr als oder gleich 20.000 und weniger als 100.000 hat, die zweite Art von abbaubarem Polyesterpolymer eine massegemittelte Molekülmasse von mehr als oder gleich 100.000 und weniger als oder gleich 1.000.000 hat, das Massenverhältnis der ersten Art von abbaubarem Polyesterpolymer zur zweiten Art von abbaubarem Polyesterpolymer im Bereich von 1:9 bis 9:1 und optional im Bereich von 1:5 bis 5:1 liegt und der gleiche Typ von abbaubarem Polyesterpolymer einer der folgenden ist: Polymilchsäure, Polyglykolsäure, Poly(butylensuccinat), Poly(beta-hydroxybutyrat), Polycaprolacton, Poly(ethylenglykoladipat), Poly(milch-co-glykolsäure) und Poly(3-hydroxybutyrat-co-3-hydroxyvalerat).

9. Resorbierbarer Stent aus einer Legierung auf Eisenbasis nach Anspruch 1, **dadurch gekennzeichnet, dass** der abbaubare Polyester mit einem Arzneimittelwirkstoff vermischt ist und das Massenverhältnis des abbaubaren Polyesters zu dem Arzneimittel im Bereich von 0,1 bis 20, optional im Bereich von 0,5 bis 10, liegt.

10. Resorbierbarer Stent aus einer Legierung auf Eisenbasis nach Anspruch 1, **dadurch gekennzeichnet, dass** das Substrat aus der Legierung auf Eisenbasis aus reinem Eisen oder einer Legierung auf Eisenbasis ausgewählt ist, die durch Dotieren von C und/oder N und/oder O und/oder S und/oder P und/oder Mn und/oder Pd und/oder Si und/oder W und/oder Ti und/oder Co und/oder Cr und/oder Cu und/oder Re in reines Eisen gebildet ist.

11. Resorbierbarer Stent aus einer Legierung auf Eisenbasis, mit einem Substrat aus einer Legierung auf Eisenbasis und einem abbaubaren Polymer in Kontakt mit der Oberfläche des Substrats, wobei das abbaubare Polymer eine massegemittelte Molekülmasse von mehr als oder gleich 20.000 und weniger als oder gleich 1.000.000 und einen Polydispersitätsindex von mehr als 1,0 und weniger als oder gleich 50 aufweist und das abbaubare Polymer in der Lage ist, eine Carboxylgruppe zu erzeugen, nachdem der Stent aus der Legierung auf Eisenbasis in den Körper implantiert ist, und wobei die Oberfläche des Substrats aus der Legierung auf Eisenbasis mit dem abbaubaren Polymer in Form einer Beschichtungslage beschichtet ist,

**dadurch gekennzeichnet, dass** dann, wenn eine Wandstärke des Substrats aus der Legierung auf Eisenbasis mehr als oder gleich 30 μm und weniger als 50 μm beträgt, eine Dicke der Beschichtungslage aus abbaubarem Polymer mehr als oder gleich 3 μm und weniger als 5 μm oder mehr als oder gleich 5 μm und weniger als 10 μm oder mehr als oder gleich 10 μm und weniger als 15 μm oder mehr als oder gleich 15 μm und weniger als oder gleich 20 μm beträgt;

oder dann, wenn die Wandstärke des Substrats aus der Legierung auf Eisenbasis mehr als oder gleich 50 μm und weniger als 100 μm beträgt, die Dicke der Beschichtungslage aus abbaubarem Polymer mehr als oder gleich 5 μm und weniger als 10 μm oder mehr als oder gleich 10 μm und weniger als 15 μm oder mehr als oder gleich 15 μm und weniger

als 20 $\mu$m oder mehr als oder gleich 20 $\mu$m und weniger als oder gleich 25 $\mu$m beträgt;

oder dann, wenn die Wandstärke des Substrats aus der Legierung auf Eisenbasis mehr als oder gleich 100 $\mu$m und weniger als 200 $\mu$m beträgt, die Dicke der Beschichtungslage aus abbaubarem Polymer mehr als oder gleich 10 $\mu$m und weniger als 15 $\mu$m oder mehr als oder gleich 15 $\mu$m und weniger als 20 $\mu$m oder mehr als oder gleich 20 $\mu$m und weniger als 25 $\mu$m oder mehr als oder gleich 25 $\mu$m und weniger als oder gleich 35 $\mu$m beträgt;

oder dann, wenn die Wandstärke des Substrats aus der Legierung auf Eisenbasis mehr als oder gleich 200 $\mu$m und weniger als oder gleich 300 $\mu$m beträgt, die Dicke der Beschichtungslage aus abbaubarem Polymer mehr als oder gleich 10 $\mu$m und weniger als 15 $\mu$m oder mehr als oder gleich 15 $\mu$m und weniger als 20 $\mu$m oder mehr als oder gleich 20 $\mu$m und weniger als 25 $\mu$m oder mehr als oder gleich 25 $\mu$m und weniger als 35 $\mu$m oder mehr als oder gleich 35 $\mu$m und weniger als oder gleich 45 $\mu$m beträgt.

12. Resorbierbarer Stent aus einer Legierung auf Eisenbasis nach Anspruch 11, **dadurch gekennzeichnet, dass** das abbaubare Polymer ein abbaubarer Polyester ist;

oder das abbaubare Polymer ein Gemisch aus dem abbaubaren Polyester und einem abbaubaren Polyanhydrid ist;

oder das abbaubare Polymer ein abbaubares Copolymer ist, das durch Copolymerisation von Monomeren gebildet ist, die den abbaubaren Polyester und das abbaubare Polyanhydrid bilden, und der abbaubare Polyester und das abbaubare Polyanhydrid eine massegemittelte Molekülmasse von mehr als oder gleich 20.000 und weniger als oder gleich 1.000.000 und Polydispersitätsindizes von mehr als 1,0 und weniger als oder gleich 50 aufweisen.

13. Resorbierbarer Stent aus einer Legierung auf Eisenbasis nach Anspruch 12, **dadurch gekennzeichnet, dass** die Polydispersitätsindizes des abbaubaren Polyesters und des abbaubaren Polyanhydrids mehr als oder gleich 1,2 und weniger als 2 oder mehr als oder gleich 2 und weniger als 3 oder mehr als oder gleich 3 und weniger als 5 oder mehr als oder gleich 5 und weniger als 10 oder mehr als oder gleich 10 und weniger als 20 oder mehr als oder gleich 20 und weniger als 30 oder mehr als oder gleich 30 und weniger als oder gleich 50 betragen.

14. Resorbierbarer Stent aus einer Legierung auf Eisenbasis nach Anspruch 12, **dadurch gekennzeichnet, dass** das Massenverhältnis des Substrats aus der Legierung auf Eisenbasis zu dem abbaubaren Polymer im Bereich von 1 bis 200, optional im Bereich von 5 bis 50 liegt.

15. Resorbierbarer Stent aus einer Legierung auf Eisenbasis nach Anspruch 12, **dadurch gekennzeichnet, dass** die massegemittelte Molekülmasse des abbaubaren Polyesters mehr als oder gleich 20.000 und weniger als 100.000 oder mehr als oder gleich 100.000 und weniger als 250.000 oder mehr als oder gleich 250.000 und weniger als 400.000 oder mehr als oder gleich 400.000 und weniger als 600.000 oder mehr als oder gleich 600.000 und weniger als 1.000.000 beträgt.

16. Resorbierbarer Stent aus einer Legierung auf Eisenbasis nach Anspruch 11, **dadurch gekennzeichnet, dass** das Substrat aus der Legierung auf Eisenbasis mit Spalten oder Rillen versehen ist und das abbaubare Polymer in den Spalten oder Rillen angeordnet ist,

oder/und das Substrat aus der Legierung auf Eisenbasis mit einem Innenhohlraum versehen ist und das abbaubare Polymer in den Innenhohlraum gefüllt ist.

17. Resorbierbarer Stent aus einer Legierung auf Eisenbasis nach Anspruch 12, **dadurch gekennzeichnet, dass** der abbaubare Polyester einer der folgenden ist: Polymilchsäure, Polyglykolsäure, Poly(butylensuccinat), Poly(beta-hydroxybutyrat), Polycaprolacton, Poly(ethylenglykoladipat), Poly(milch-co-glykolsäure) und Poly(3-hydroxybutyrat-co-3-hydroxyvalerat);

oder der abbaubare Polyester mindestens zwei Arten des gleichen Typs von abbaubaren Polyesterpolymeren umfasst, wobei die erste Art von abbaubarem Polyesterpolymer eine massegemittelte Molekülmasse von mehr als oder gleich 20.000 und weniger als 100.000 hat, die zweite Art von abbaubarem Polyesterpolymer eine massegemittelte Molekülmasse von mehr als oder gleich 100.000 und weniger als oder gleich 1.000.000 hat, das Massenverhältnis der ersten Art von abbaubarem Polyesterpolymer zur zweiten Art von abbaubarem Polyesterpolymer mehr als oder gleich 1:9 und weniger als oder gleich 9:1 ist und der gleiche Typ von abbaubarem Polyesterpolymer einer der folgenden ist: Polymilchsäure, Polyglykolsäure, Poly(butylensuccinat), Poly(beta-hydroxybutyrat), Polycaprolacton, Poly(ethylenglykoladipat), Poly(milch-co-glykolsäure) und Poly(3-hydroxybutyrat-co-3-hydroxyvalerat);

oder der abbaubare Polyester mindestens zwei Arten von abbaubaren Polyesterpolymeren mit hoher relativer Molekülmasse umfasst und jede der mindestens zwei Arten von abbaubaren Polyesterpolymeren mit hoher relativer Molekülmasse eine massegemittelte Molekülmasse von mehr als oder gleich 100.000 und weniger als 200.000 oder mehr als oder gleich 200.000 und weniger als 400.000 oder mehr als oder gleich 400.000 und weniger als 600.000 oder mehr als oder gleich 600.000 und weniger als oder gleich 1.000.000 hat;

oder der abbaubare Polyester ein Gemisch aus mindestens zweien der folgenden ist: Polymilchsäure, Polyglykolsäure, Poly(butylensuccinat), Poly(beta-hydroxybutyrat), Polycaprolacton, Poly(ethylenglykoladipat), Poly(milch-co-glykolsäure) und Poly(3-hydroxybutyrat-co-3-hydroxyvalerat), oder ein Copolymer ist, das durch Copolymerisation von mindestens zweien aus Monomeren, die Polymilchsäure, Polyglykolsäure, Poly(butylensuccinat), Poly(beta-hydroxybutyrat), Polycaprolacton, Poly(ethylenglykoladipat), Poly(milch-co-glykolsäure) und Poly(3-hydroxybutyrat-co-3-hydroxyvalerat) bilden, gebildet ist;

oder der abbaubare Polyester eine Mischung aus mindestens zwei Arten von abbaubaren Polyesterpolymeren mit unterschiedlicher Kristallinität ist, wobei der Gehalt an abbaubarem Polyesterpolymer mit einer Kristallinität im Bereich von 5% bis 50% im Bereich von 10% bis 90% in Massenprozent liegt und das abbaubare Polyesterpolymer aus der aus Polymilchsäure, Polyglykolsäure, Poly(butylensuccinat), Poly(beta-hydroxybutyrat), Polycaprolacton, Poly(ethylenglykoladipat), Poly(milch-co-glykolsäure) und Poly(3-hydroxybutyrat-co-3-hydroxyvalerat) bestehenden Gruppe ausgewählt ist.

18. Resorbierbarer Stent aus einer Legierung auf Eisenbasis nach Anspruch 17, **dadurch gekennzeichnet, dass** dann, wenn der abbaubare Polyester mindestens zwei Arten des gleichen Typs von abbaubaren Polyesterpolymeren umfasst, das Massenverhältnis der ersten Art von abbaubarem Polyesterpolymer zur zweiten Art von abbaubarem Polyesterpolymer im Bereich von 1:5 bis 5:1 liegt.

19. Resorbierbarer Stent aus einer Legierung auf Eisenbasis nach Anspruch 12, **dadurch gekennzeichnet, dass** das Polyanhydrid eines der folgenden ist: Poly(1,3-bis(p-carboxyphenoxy)propansebacinsäure) und Poly(erucasäure-dimer-sebacinsäure) oder Poly(fumarsäure-sebacinsäure), der abbaubare Polyester einer der folgenden ist: Polymilchsäure, Polyglykolsäure, Poly(butylensuccinat), Poly(beta-hydroxybutyrat), Polycaprolacton, Poly(ethylenglykoladipat), Poly(milch-co-glykolsäure) und Poly(3-hydroxybutyrat-co-3-hydroxyvalerat), und das Massenverhältnis des abbaubaren Polyesters zum Polyanhydrid im Bereich von 1:9 bis 9:1 liegt.

20. Resorbierbarer Stent aus einer Legierung auf Eisenbasis nach Anspruch 12, **dadurch gekennzeichnet, dass** in dem Gemisch aus dem abbaubaren Polyester und dem abbaubaren Polyanhydrid der Gehalt an abbaubarem Polyester oder abbaubarem Polyanhydrid mit einer Kristallinität im Bereich von 5% bis 50% im Bereich von 10% bis 90% in Massenprozent liegt, das abbaubare Polyesterpolymer aus der aus Polymilchsäure, Polyglykolsäure, Poly(butylensuccinat), Poly(beta-hydroxybutyrat), Polycaprolacton, Poly(ethylenglykoladipat), Poly(milch-co-glykolsäure) und Poly(3-hydroxybutyrat-co-3-hydroxyvalerat) bestehenden Gruppe ausgewählt ist und das Polyanhydrid aus der aus Poly(1,3-bis(p-carboxyphenoxy)propan-sebacinsäure) und Poly(erucasäure-dimer-sebacinsäure) oder Poly(fumarsäure-sebacinsäure) bestehenden Gruppe ausgewählt ist.

21. Resorbierbarer Stent aus einer Legierung auf Eisenbasis nach Anspruch 11, **dadurch gekennzeichnet, dass** das abbaubare Polymer mit einem Arzneimittelwirkstoff vermischt ist und das Massenverhältnis des abbaubaren Polymers zu dem Arzneimittel im Bereich von 0,1 bis 20, optional im Bereich von 0,5 bis 10, liegt.

22. Resorbierbarer Stent aus einer Legierung auf Eisenbasis nach Anspruch 11, **dadurch gekennzeichnet, dass** das Substrat aus der Legierung auf Eisenbasis aus reinem Eisen oder einer Legierung auf Eisenbasis ausgewählt ist, die durch Dotieren von C und/oder N und/oder O und/oder S und/oder P und/oder Mn und/oder Pd und/oder Si und/oder W und/oder Ti und/oder Co und/oder Cr und/oder Cu und/oder Re in reines Eisen gebildet ist.

**Revendications**

1. Stent absorbable en alliage à base de fer, comprenant un substrat en alliage à base de fer et un polyester dégradable en contact avec la surface du substrat, le polyester dégradable présentant une masse moléculaire moyenne en poids dans la plage de 20 000 à 1 000 000 et un indice de polydispersité supérieur ou égal à 1,2 et inférieur ou égal à 30, ou supérieur à 1,0 et inférieur à 1,2, ou supérieur à 30 et inférieur ou égal à 50, et dans lequel la surface du substrat en alliage à base de fer est revêtue de polyester dégradable sous la forme d'une couche de revêtement,

**caractérisé en ce que**

lorsqu'une épaisseur de paroi du substrat en alliage à base de fer est supérieure ou égale à 30 $\mu$m et inférieure à 50 $\mu$m, une épaisseur de la couche de revêtement en polyester dégradable est supérieure ou égale à 3 $\mu$m et inférieure à 5 $\mu$m, ou supérieure ou égale à 5 $\mu$m et inférieure à 10 $\mu$m, ou supérieure ou égale à 10 $\mu$m et inférieure à 15 $\mu$m, ou supérieure ou égale à 15 $\mu$m et inférieure ou égale à 20 $\mu$m ; ou lorsque l'épaisseur de paroi du substrat en alliage à base de fer est supérieure ou égale à 50 $\mu$m et inférieure à 100 $\mu$m, l'épaisseur de la couche de revêtement en polyester dégradable est supérieure ou égale à 5 $\mu$m et inférieure à 10 $\mu$m, ou supérieure ou égale à 10 $\mu$m et inférieure à 15 $\mu$m, ou supérieure

ou égale à 15 μm et inférieure à 20 μm, ou supérieure ou égale à 20 μm et inférieure ou égale à 25 μm ; ou lorsque l'épaisseur de paroi du substrat en alliage à base de fer est supérieure ou égale à 100 μm et inférieure à 200 μm, l'épaisseur de la couche de revêtement en polyester dégradable est supérieure ou égale à 10 μm et inférieure à 15 μm, ou supérieure ou égale à 15 μm et inférieure à 20 μm, ou supérieure ou égale à 20 μm et inférieure à 25 μm, ou supérieure ou égale à 25 μm et inférieure ou égale à 35 μm ; ou lorsque l'épaisseur de paroi du substrat en alliage à base de fer est supérieure ou égale à 200 μm et inférieure ou égale à 300 μm, l'épaisseur de la couche de revêtement en polyester dégradable est supérieure ou égale à 10 μm et inférieure à 15 μm, ou supérieure ou égale à 15 μm et inférieure à 20 μm, ou supérieure ou égale à 20 μm et inférieure à 25 μm, ou supérieure ou égale à 25 μm ou inférieure à 35 μm, ou supérieure ou égale à 35 μm et inférieure ou égale à 45 μm.

2. Stent absorbable en alliage à base de fer selon la revendication 1, **caractérisé en ce que** la masse moléculaire moyenne en poids du polyester dégradable est supérieure ou égale à 20 000 et inférieure à 100 000, ou supérieure ou égale à 100 000 et inférieure à 250 000, ou supérieure ou égale à 250 000 et inférieure à 400 000, ou supérieure ou égale à 400 000 et inférieure à 600 000, ou supérieure ou égale à 600 000 et inférieure ou égale à 1 000 000.

3. Stent absorbable en alliage à base de fer selon la revendication 1, **caractérisé en ce que** l'indice de polydispersité est supérieur ou égal à 1,2 et inférieur à 2, ou supérieur ou égal à 2 et inférieur à 3, ou supérieur ou égal à 3 et inférieur à 5, ou supérieur ou égal à 5 et inférieur à 10, ou supérieur ou égal à 10 et inférieur à 20, ou supérieur ou égal à 20 et inférieur ou égal à 30.

4. Stent absorbable en alliage à base de fer selon la revendication 1, **caractérisé en ce que** le rapport de masse du substrat en alliage à base de fer au polyester dégradable est supérieur ou égal à 1 et inférieur ou égal à 200.

5. Stent absorbable en alliage à base de fer selon la revendication 4, **caractérisé en ce que** le rapport de masse du substrat en alliage à base de fer au polyester dégradable est supérieur ou égal à 5 et inférieur ou égal à 50.

6. Stent absorbable en alliage à base de fer selon la revendication 1, **caractérisé en ce que** le substrat en alliage à base de fer est pourvu de fentes ou de rainures et le polyester dégradable est agencé dans les fentes ou les rainures ; ou/et le substrat en alliage à base de fer est pourvu d'une cavité interne et le polyester dégradable est rempli dans la cavité interne.

7. Stent absorbable en alliage à base de fer selon la revendication 1, **caractérisé en ce que** le polyester dégradable est l'un des suivants : acide polylactique, acide polyglycolique, poly(succinate de butylène), poly(bêta-hydroxybutyrate), polycaprolactone, poly(adipate d'éthylèneglycol), poly(acide lactique-co-glycolique) et poly(3-hydroxybutyrate-co-3-hydroxyvalérate) ; ou
le polyester dégradable comprend au moins deux genres de polymères de polyester dégradables à poids moléculaire élevé, chacun des deux genres de polymères de polyester dégradables à poids moléculaire élevé présentant une masse moléculaire moyenne en poids supérieure ou égale à 100 000 et inférieure à 200 000, ou supérieure ou égale à 200 000 et inférieure à 400 000, ou supérieure ou égale à 400 000 et inférieure à 600 000, ou supérieure ou égale à 600 000 et inférieure ou égale à 1 000 000 ; ou
le polyester dégradable est un mélange d'au moins deux des éléments suivants : acide polylactique, acide polyglycolique, poly(succinate de butylène), poly(bêta-hydroxybutyrate), polycaprolactone, poly(adipate d'éthylène glycol), poly(acide lactique-co-glycolique) et poly(3-hydroxybutyrate-co-3-hydroxyvalérate), ou un copolymère obtenu par copolymérisation d'au moins deux des monomères qui forment de l'acide polylactique, de l'acide polyglycolique, du poly(succinate de butylène), du poly(bêta-hydroxybutyrate), de la polycaprolactone, du poly(adipate d'éthylène glycol), du poly(acide lactique-co-glycolique) et du poly(3-hydroxybutyrate-co-3-hydroxyvalérate) ; ou
le polyester dégradable est un mélange d'au moins deux genres de polymères de polyester dégradables présentant une cristallinité différente, la teneur en polymère de polyester dégradable présentant une cristallinité supérieure ou égale à 5% et inférieure ou égale à 50% étant supérieure ou égale à 10% et inférieure ou égale à 90% en masse, et le polymère de polyester dégradable étant choisi dans le groupe constitué d'acide polylactique, d'acide polyglycolique, de poly(succinate de butylène), de poly(bêta-hydroxybutyrate), de polycaprolactone, de poly(adipate d'éthylène glycol), de poly(acide lactique-co-glycolique) et de poly(3-hydroxybutyrate-co-3-hydroxyvalérate).

8. Stent absorbable en alliage à base de fer selon la revendication 1, **caractérisé en ce que** le polyester dégradable comprend au moins deux genres du même type de polymères de polyester dégradables, le premier genre de polymère de polyester dégradable présentant une masse moléculaire moyenne en poids supérieure ou égale à 20 000 et inférieure à 100 000, le deuxième genre de polymère de polyester dégradable présentant une masse moléculaire moyenne en poids supérieure ou égale à 100 000 et inférieure ou égale à 1 000 000, le rapport de masse du premier genre de polymère de polyester dégradable au deuxième genre de polymère de polyester dégradable étant compris dans la plage de 1:9 à 9:1 et, en option, dans la plage de 1:5 à 5:1, et le même genre de polymère de polyester dégradable étant l'un des suivants : acide polylactique, acide polyglycolique, poly(succinate de butylène), poly(bêta-hydroxybutyrate), polycaprolactone, poly(adipate d'éthylène glycol), poly(acide lactique-co-glycolique) et poly(3-hydroxybutyrate-co-3-hydroxyvalérate).

9. Stent absorbable en alliage à base de fer selon la revendication 1, **caractérisé en ce que** le polyester dégradable est mélangé avec un médicament actif et le rapport de masse du polyester dégradable au médicament est dans la plage de 0,1 à 20, en option dans la plage de 0,5 à 10.

10. Stent absorbable en alliage à base de fer selon la revendication 1, **caractérisé en ce que** le substrat en alliage à base de fer est choisi parmi un alliage à base de fer de fer pur ou un alliage à base de fer réalisé par dopage de C et/ou N et/ou O et/ou S et/ou P et/ou Mn et/ou Pd et/ou Si et/ou W et/ou Ti et/ou Co et/ou Cr et/ou Cu et/ou Re en fer pur.

11. Stent absorbable en alliage à base de fer, comprenant un substrat en alliage à base de fer et un polymère dégradable en contact avec la surface du substrat, le polymère dégradable présentant une masse moléculaire moyenne en poids supérieure ou égale à 20 000 et inférieure ou égale à 1 000 000 et un indice de polydispersité supérieur à 1,0 et inférieur ou égal à 50, le polymère dégradable étant apte à générer un groupe carboxyle après l'implantation du stent en alliage à base de fer dans le corps, et dans lequel la surface du substrat en alliage à base de fer est revêtue du polymère dégradable sous la forme d'une couche de revêtement, **caractérisé en ce que** lorsqu'une épaisseur de paroi du substrat en alliage à base de fer est supérieure ou égale à 30 $\mu$m et inférieure à 50 $\mu$m, une épaisseur de la couche de revêtement en polymère dégradable est supérieure ou égale à 3 $\mu$m et inférieure à 5 $\mu$m, ou supérieure ou égale à 5 $\mu$m et inférieure à 10 $\mu$m, ou supérieure ou égale à 10 $\mu$m et inférieure à 15 $\mu$m, ou supérieure ou égale à 15 $\mu$m et inférieure ou égale à 20 $\mu$m ; ou lorsque l'épaisseur de paroi du substrat en alliage à base de fer est supérieure ou égale à 50 $\mu$m et inférieure à 100 $\mu$m, l'épaisseur de la couche de revêtement en polymère dégradable est supérieure ou égale à 5 $\mu$m et inférieure à 10 $\mu$m, ou supérieure

ou égale à 10 $\mu$m et inférieure à 15 $\mu$m, ou supérieure ou égale à 15 $\mu$m et inférieure à 20 $\mu$m, ou supérieure ou égale à 20 $\mu$m et inférieure ou égale à 25 $\mu$m ; ou lorsque l'épaisseur de paroi du substrat en alliage à base de fer est supérieure ou égale à 100 $\mu$m et inférieure à 200 $\mu$m, l'épaisseur de la couche de revêtement de polymère dégradable est supérieure ou égale à 10 $\mu$m et inférieure à 15 $\mu$m, ou supérieure ou égale à 15 $\mu$m et inférieure à 20 $\mu$m, ou supérieure ou égale à 20 $\mu$m et inférieure à 25 $\mu$m, ou supérieure ou égale à 25 $\mu$m et inférieure ou égale à 35 $\mu$m ; ou lorsque l'épaisseur de paroi du substrat en alliage à base de fer est supérieure ou égale à 200 $\mu$m et inférieure ou égale à 300 $\mu$m, l'épaisseur de la couche de revêtement en polymère dégradable est supérieure ou égale à 10 $\mu$m et inférieure à 15 $\mu$m, ou supérieure ou égale à 15 $\mu$m et inférieure à 20 $\mu$m, ou supérieure ou égale à 20 $\mu$m et inférieure à 25 $\mu$m, ou supérieure ou égale à 25 $\mu$m et inférieure à 35 $\mu$m, ou supérieure ou égale à 35 $\mu$m et inférieure ou égale à 45 $\mu$m.

12. Stent absorbable en un alliage à base de fer selon la revendication 11, **caractérisé en ce que** le polymère dégradable est un polyester dégradable ; ou le polymère dégradable est un mélange du polyester dégradable et d'un polyanhydride dégradable ; ou le polymère dégradable est un copolymère dégradable réalisé par copolymérisation de monomères formant le polyester dégradable et le polyanhydride dégradable, et le polyester dégradable et le polyanhydride dégradable présentent une masse moléculaire moyenne en poids supérieure ou égale à 20 000 et inférieure ou égale à 1 000 000 et des indices de polydispersité supérieurs à 1,0 et inférieurs ou égaux à 50.

13. Stent absorbable en alliage à base de fer selon la revendication 12, **caractérisé en ce que** les indices de polydispersité du polyester dégradable et du polyanhydride dégradable sont supérieurs ou égaux à 1,2 et inférieurs à 2, ou supérieurs ou égaux à 2 et inférieurs à 3, ou supérieurs ou égaux à 3 et inférieurs à 5, ou supérieurs ou égaux à 5 et inférieurs à 10, ou supérieurs ou égaux à 10 et inférieurs à 20, ou supérieurs ou égaux à 20 et inférieurs à 30, ou supérieurs ou égaux à 30 et inférieurs ou égaux à 50.

14. Stent absorbable en alliage à base de fer selon la revendication 12, **caractérisé en ce que** le rapport de masse du substrat en alliage à base de fer au polymère dégradable est dans la plage de 1 à 200, en option dans la plage de 5 à 50.

15. Stent absorbable en alliage à base de fer selon la revendication 12, **caractérisé en ce que** la masse moléculaire moyenne en poids du polyester dégradable est supérieure ou égale à 20 000 et inférieure à 100 000, ou supérieure ou égale à 100 000 et inférieure à 250 000, ou supérieure ou égale à 250 000 et inférieure à 400 000, ou supérieure ou égale à 400 000 et inférieure à 600 000, ou supérieure ou égale à 600 000 et inférieure à 1 000 000.

16. Stent absorbable en alliage à base de fer selon la revendication 11, **caractérisé en ce que** le substrat en alliage à base de fer est pourvu de fentes ou de rainures et le polymère dégradable est agencé dans les fentes ou les rainures ;
ou/et le substrat en alliage à base de fer est pourvu d'une cavité interne et le polymère dégradable est rempli dans la cavité interne.

17. Stent absorbable en alliage à base de fer selon la revendication 12, **caractérisé en ce que** le polyester dégradable est l'un des suivants : acide polylactique, acide polyglycolique, poly(succinate de butylène), poly(bêta-hydroxybutyrate), polycaprolactone, poly(adipate d'éthylène glycol), poly(acide lactique-co-glycolique) et poly(3-hydroxybutyrate-co-3-hydroxyvalérate) ;
ou le polyester dégradable comprend au moins deux genres du même type de polymères de polyester dégradables, le premier genre de polymère de polyester dégradable présentant une masse moléculaire moyenne en poids supérieure ou égale à 20 000 et inférieure à 100 000, le deuxième genre de polymère de polyester dégradable présentant une masse moléculaire moyenne en poids supérieure ou égale à 100 000 et inférieure ou égale à 1 000 000, le rapport de masse du premier genre de polymère de polyester dégradable au deuxième genre de polymère de polyester dégradable étant supérieur ou égal à 1:9 et inférieur ou égal à 9:1, et le même type de polymère de polyester dégradable étant l'un des suivants : acide polylactique, acide polyglycolique, poly(succinate de butylène), poly(bêta-hydroxybutyrate), polycaprolactone, poly(adipate d'éthylène glycol), poly(acide lactique-co-glycolique) et poly(3-hydroxybutyrate-co-3-hydroxyvalérate) ;
ou le polyester dégradable comprend au moins deux genres de polymères de polyester dégradables à poids moléculaire élevé, et chacun desdits au moins deux genres de polymères de polyester dégradables à poids moléculaire élevé présente une masse moléculaire moyenne en poids supérieure ou égale à 100 000 et inférieure à 200 000, ou supérieure ou égale à 200 000 et inférieure à 400 000, ou supérieure ou égale à 400 000 et inférieure à 600 000, ou supérieure ou égale à 600 000 et inférieure ou égale à 1 000 000 ;
ou le polyester dégradable est un mélange d'au moins deux des éléments suivants : acide polylactique, acide polyglycolique, poly(succinate de butylène), poly(bêta-hydroxybutyrate), polycaprolactone, poly(adipate d'éthylène glycol), poly(acide lactique-co-glycolique) et poly(3-hydroxybutyrate-co-3-hydroxyvalérate), ou un copolymère obtenu par copolymérisation d'au moins deux de monomères qui forment de l'acide polylactique, de l'acide polyglycolique, du poly(succinate de butylène), du poly(bêta-hydroxybutyrate), de la polycaprolactone, du poly(adipate d'éthylène glycol), du poly(acide lactique-co-glycolique) et du poly(3-hydroxybutyrate-co-3-hydroxyvalérate) ;
ou le polyester dégradable est un mélange d'au moins deux genre de polymères de polyester dégradables présentant une cristallinité différente, la teneur en polymère de polyester dégradable présentant une cristallinité dans la plage de 5% à 50% étant dans la plage de 10% à 90% en masse et le polymère de polyester dégradable étant choisi dans le groupe constitué d'acide polylactique, d'acide polyglycolique, de poly(succinate de butylène), de poly(bêta-hydroxybutyrate), de polycaprolactone, de poly(adipate d'éthylène glycol), de poly(acide lactique-co-glycolique) et de poly(3-hydroxybutyrate-co-3-hydroxyvalérate).

18. Stent absorbable en alliage à base de fer selon la revendication 17, **caractérisé en ce que** lorsque le polyester dégradable comprend au moins deux genres du même type de polymères de polyester dégradables, le rapport de masse du premier genre de polymère de polyester dégradable au deuxième genre de polymère de polyester dégradable est dans la plage de 1:5 à 5:1.

19. Stent absorbable en alliage à base de fer selon la revendication 12, **caractérisé en ce que** le polyanhydride est l'un des suivants : poly(1,3-bis(p-carboxyphénoxy)propane acide sébacique) et poly(acide érucique-dimère acide sébacique) ou poly(acide fumarique-sébacique), le polyester dégradable est l'un des suivants : acide polylactique, acide polyglycolique, poly(butylène succinate), poly(bêta-hydroxybutyrate), polycaprolactone, poly(éthylène glycol adipate), poly(acide lactique-co-glycolique) et poly(3-hydroxybutyrate-co-3-hydroxyvalérate), et le rapport de masse du polyester dégradable au polyanhydride est compris dans la plage de 1:9 à 9:1.

20. Stent absorbable en alliage à base de fer selon la revendication 12, **caractérisé en ce que** dans le mélange du polyester dégradable et du polyanhydride dégradable, la teneur en polyester dégradable ou en polyanhydride dégradable présentant une cristallinité dans la plage de 5% à 50% est dans la plage de 10% à 90% en masse, le polymère de polyester dégradable est choisi dans le groupe constitué d'acide polylactique, d'acide polyglycolique, de poly(succinate de butylène), de poly(bêta-hydroxybutyrate), de polycaprolactone, de poly(adipate d'éthylène glycol), de poly(acide lactique-co-glycolique) et de poly(3-hydroxybutyrate-co-3-hydroxyvalérate), et le polyanhydride est choisi dans le groupe constitué de poly(l, 3-bis(p-carboxyphénoxy)propane acide sébacique) et de poly(acide érucique dimère acide sébacique) ou de poly(acide fumarique acide sébacique).

21. Stent absorbable en alliage à base de fer selon la revendication 11, **caractérisé en ce que** le polymère dégradable est mélangé avec un médicament actif et le rapport de masse du polymère dégradable au médicament est dans la plage de 0,1 à 20, en option dans la plage de 0,5 à 10.

22. Stent absorbable en alliage à base de fer selon la revendication 11, **caractérisé en ce que** le substrat en alliage à base de fer est choisi parmi un alliage à base de fer de fer pur ou un alliage à base de fer réalisé par dopage de C et/ou N et/ou O et/ou S et/ou P et/ou Mn et/ou Pd et/ou Si et/ou W et/ou Ti et/ou Co et/ou Cr et/ou Cu et/ou Re en fer pur.

Fig. 1

Fig. 2

Fig. 3

Fig. 4

Fig. 5

Fig. 6

Fig. 7

**EP 3 064 232 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102228721